# FASCICULE DE BREVET EUROPEEN

(11) **EP 2 276 826 B1**
(45) Date de publication et mention de la délivrance du brevet: **13.09.2017**
(21) Numéro de dépôt: 09754056.1
(22) Date de dépôt: 10.04.2009
(51) Int. Cl.: C12F 3/06, C12H 1/04, A61K 8/97, A61Q 19/08, A61K 36/73, A61K 36/8998, A23L 33/105

(54) **PROCÉDÉ D'OBTENTION D'EXTRAITS CONCENTRÉS EN POLYPHÉNOLS ISSUS DU PROCÉDÉ DE BRASSAGE**
VERFAHREN ZUR GEWINNUNG KONZENTRIERTER POLYPHENOLEXTRAKTE AUS EINEM BRAUVERFAHREN
METHOD FOR OBTAINING CONCENTRATED POLYPHENOL EXTRACTS FROM A BREWING PROCESS

(30) Priorité: 11.04.2008 EP 08290357
(43) Date de publication de la demande: 26.01.2011
(73) Titulaire: BRASSERIES KRONENBOURG, 67200 Strasbourg (FR)
(72) Inventeur: TAIDI, Behnam, F-67000 Strasbourg (FR); DORIAT, Jean-François, F-54840 Velaine-en-Haye (FR); MALPOTE, Jean-Yves, F-67370 Griesheim Sur Souffel (FR)
(74) Mandataire: Gevers & Orès
(86) Numéro de dépôt international: PCT/FR2009/000417
(87) Numéro de publication internationale: WO 2009/144406

(56) Documents cités:
- WO-A1-2008/090460
- DE-A1-102007 001 349
- JP-A- 2007 039 397
- US-A- 4 910 182
- US-A1- 2005 191 268
- US-A1- 2007 254 063
- GERHÄUSER ET AL.: "Isolation and potential cancer chemopreventive activities of phenolic compounds of beer", PHYTOCHEMISTRY REVIEWS, vol. 1, no. 3, 2002, pages 369-377, XP002495483,
- TEDESCO ET AL.: "Antioxidant and Cytotoxic Properties of Lyophilized Beer Extratcs on HL-60 Cell Line", NUTRITION AND CANCER, vol. 52, no. 1, 2005, pages 74-83, XP008096314,
- GASOWSKI B ET AL: "The influence of beer with different antioxidant potential on plasma lipids, plasma antioxidant capacity, and bile excretion of rats fed cholesterol-containing and cholesterol-free diets", JOURNAL OF NUTRITIONAL BIOCHEMISTRY, BUTTERWORTH PUBLISHERS, STONEHAM, GB, vol. 15, no. 9, 1 septembre 2004 (2004-09-01), pages 527-533, XP004553421, ISSN: 0955-2863
- CALLEMIEN D ET AL: "Use of thiolysis hyphenated to RP-HPLC-ESI(-)-MS/MS for the analysis of flavanoids in fresh lager beers", FOOD CHEMISTRY, ELSEVIER SCIENCE PUBLISHERS LTD, GB, vol. 110, no. 4, 15 octobre 2008 (2008-10-15), pages 1012-1018, XP022668405, ISSN: 0308-8146 [extrait le 2008-03-05]
- SKOVENBORG, ERIK: "Red Wine Polyphenols in Cancer Research", , [Online] 13 novembre 2007 (2007-11-13), XP002495489, Extrait de l'Internet: URL:http://web.archive.org/web/20071113002 457/http://www.aim-digest.com/gateway/page s/cancer/articles/polyph.htm> [extrait le 2008-09-10]
- DVORAKOVA M ET AL: "Determination of polyphenols in beer by an effective method based on solid-phase extraction and high performance liquid chromatography with diode-array detection", CZECH JOURNAL OF FOOD SCIENCE, PRAGUE, CZ, vol. 25, no. 4, 1 January 2007 (2007-01-01), pages 182-188, XP008100846, ISSN: 1212-1800

## Description

La présente invention concerne le domaine de la valorisation des co-produits de l'industrie brassicole. En particulier, l'invention propose des procédés pour obtenir des extraits concentrés en polyphénols issus du procédé de brassage, ainsi que plusieurs applications de ces extraits, qui présentent des propriétés remarquables.

La bière est fabriquée à partir de céréales (essentiellement de l'orge, à laquelle peuvent être adjointes d'autres céréales telles que le blé, le riz ou le maïs), qui subissent différents traitements. La fabrication de la bière comprend essentiellement les étapes suivantes :

### - Maltage :

Cette étape sert à activer des enzymes de l'orge, qui transformeront l'amidon en sucres au cours du brassage. L'orge, céréale dure et riche en amidon insoluble, est transformée en malt friable et riche en composés solubles, par la succession des trois étapes suivantes : le trempage, au cours duquel le grain se gorge d'eau, la germination, et le touraillage, qui stoppe la germination.

### - Brassage :

Le malt concassé est mélangé à 2 à 3 fois son volume d'eau dans des cuves appelées "cuves matières"; cette opération est appelée "empâtage". Après cuisson à différents paliers de température pendant 2 à 6 heures, le malt devient moût. Lors de cette opération, l'amidon contenu dans le malt se transforme en sucre.

La même opération peut être réalisée parallèlement dans une autre cuve si on utilise une céréale non maltée (grains crus).

Le tout est ensuite mélangé pour constituer la maïsche. Filtrée une première fois et débarrassée des enveloppes du grain dans la cuve-filtre, la maïsche devient "bouillon". L'ensemble est alors transféré dans la chaudière à houblonner où le moût est cuit à 100°C pendant 1 à 2 heures et additionné de houblon à raison de 1 à 2 g par litre. Le houblon apporte amertume et arômes à la bière et facilite sa conservation grâce à ses propriétés antiseptiques.

Par extension, on désigne souvent par "brassage" l'ensemble du procédé de fabrication de la bière (comprenant la fermentation).

### - Fermentation :

Refroidi dans un échangeur à la température de fermentation souhaitée, le moût est ensemencé avec la levure qui transformera le sucre en alcool, en arômes et en gaz carbonique. La fermentation dure quelques semaines et est réalisée dans de grandes cuves de fermentation.

Selon le type de bière recherché, les levures utilisées sont différentes ; pour les bières de type Ale, on utilise des levures de fermentation haute de type *Saccharomyces cerevisisae* alors que pour les bières de type Lager, on utilise des souches de fermentation basse de type *Saccharomyces uvarum.*

### - Garde :

Le mélange est ensuite filtré pour donner la "bière verte" qui est placée dans des cuves de garde à une température de 0°C. Elle y mûrit pendant plusieurs semaines pour donner naissance à la bière de garde. Pendant cette phase, la bière s'affine lentement et acquiert son bouquet et son goût suite au travail de la levure et du froid (*N.b.* : Cette deuxième fermentation n'est pas effectuée systématiquement en brasserie moderne).

### - Filtration :

La filtration vise à améliorer la limpidité et la brillance de la bière en éliminant les dernières levures et les particules colloïdales encore en suspension. La bière est filtrée sur des filtres kieselguhr, poudre minérale formée de diatomées (micro-organismes marins) fossilisées.

Cette étape de filtration n'est pas suffisante. En effet, même après cette filtration, un trouble de la bière peut se former au froid et devenir permanent avec le stockage de la bière. Ce trouble est formé par l'association de polyphénols avec des protéines, ce qui conduit à la formation de composés insolubles. Pour contourner ce problème, les polyphénols indésirables peuvent être retirés de la bière par passage sur une résine : la PVPP (polyvinylpolypyrrolidone). La PVPP est un poly-[1-(2-oxo-1-pyrrolidinyl)-éthylène] réticulé de façon aléatoire. Elle est produite par polymérisation de la N-vinyl-2-pyrrolidone en présence d'un catalyseur caustique ou d'une N, N'-divinyl-imidazolidone. Sa formule chimique est (C₆H₉NO)ₙ. A l'heure actuelle, elle est régénérée par lavage à la soude et les effluents partent à l'égout.

Les polyphénols (ou composés phénoliques) sont des molécules spécifiques du règne végétal. L'élément structural de base est un noyau benzénique auquel sont directement liés un ou plusieurs groupements hydroxyles-libres ou engagés dans une autre fonction chimique. Plus de 8000 composés répondent à cette définition. Selon leurs caractéristiques, ils se répartissent en différentes familles : les acides phénoliques, les flavonoïdes, les tanins et les lignanes qui, avec les isoflavones, sont nommés phyto-oestrogènes. Ces composés sont utilisés dans l'industrie, en particulier pour leurs propriétés colorantes et anti-oxydantes.

Dans ce contexte, les inventeurs ont étudié la possibilité de récupérer les polyphénols éliminés de la bière à la fin de sa fabrication. Du fait de leurs propriétés biologiques générales (anti-oxydantes, biocides, anti-inflammatoires...), les polyphénols sont en effet susceptibles de présenter un intérêt dans différents domaines :
- cosmétique
- agro-alimentaire / nutraceutique
- phytothérapie
- médecine vétérinaire
- pharmacie.

Les extraits de polyphénols actuellement disponibles sur le marché sont issus des composés végétaux suivants : olives, café, cacao, thé, raisin, vin, pomme, soja, algue, cassis ou écorce de pin.

La préparation d'extraits riches en polyphénols après traitement de la bière a été décrite (Gerhäuser et al., Phytochemistry Reviews, 1 : 369-377, 2002). A partir d'une résine PVPP adsorbant les polyphénols, une étape d'extraction est réalisée en présence d'acétate d'éthyle. Après évaporation du solvant, le résidu obtenu est dissous dans un mélange MeOH/Cl₂CH₂ puis séparé sur une colonne hydrophile et lipophile (Sephadex LH 20). Le fractionnement est réalisé par chromatographie en phase liquide sous vide (VLC) et la séparation finale est obtenue par HPLC.

Les inventeurs ont également étudié l'intérêt des polyphénols extraits du procédé de fabrication de la bière, après l'étape de fermentation, dans diverses applications. En effet, ces composés phénoliques ont subi l'impact du maltage, du brassage et de la fermentation, ce qui a vraisemblablement entraîné des modifications de leur structure (liaison à des protéines, à des polysaccharides, oxydation et polymérisation partielles). Ces modifications risquent d'influer sur leurs propriétés, notamment sur leurs propriétés anti-oxydantes. De manière surprenante, les inventeurs ont démontré que les polyphénols issus du procédé de brassage (pris au sens étendu) présentent d'excellentes propriétés anti-oxydantes.

La présente invention porte donc en premier lieu sur l'utilisation d'une bière partiellement purifiée, comme matière première pour l'obtention d'un extrait riche en polyphénols. Par "bière partiellement purifiée", on entend ici la bière obtenue après élimination des levures, débris végétaux et particules colloïdales, par exemple par filtration sur des filtres kieselguhr (d'autres procédés, notamment basés sur la centrifugation, ont également été décrits à cette fin). Un "extrait riche en polyphénols", ou "extrait de polyphénols", ou "concentré de polyphénols" désigne ici un extrait végétal sous une forme quelconque (liquide ou solide) dont la teneur en polyphénols totaux est d'au moins 5%, de préférence au moins 10 % pour la forme liquide, et d'au moins 50%, de préférence au moins 60 à 80% ou même davantage pour la forme solide. La méthode Folin-Ciocalteu (standard EBC) peut par exemple être utilisée pour déterminer la teneur en polyphénols dans les concentrés selon l'invention (Colin and Ciocalteu, 1927).

Un procédé d'obtention d'un extrait de polyphénols issus du procédé de brassage fait également partie de la présente invention. Il comprend au moins les étapes suivantes :
(i) mise en contact d'une bière partiellement purifiée, obtenue après élimination des levures, débris végétaux et particules colloïdales, avec une résine adsorbant les polyphénols ;
(ii) récupération des polyphénols adsorbés sur ladite résine. Bien entendu, l'expression "procédé de brassage" doit ici être comprise dans son sens étendu, désignant le procédé de fabrication de la bière. Un exemple de résine utilisable pour mettre en oeuvre ce procédé est la PVPP.

L'étape de "récupération" des polyphénols adsorbés sur la résine après son contact avec la bière partiellement purifiée implique que ces polyphénols soient désorbés de ladite résine, placés dans des conditions garantissant leur stabilité et, le cas échéant, concentrés et/ou purifiés. Dans le cadre de la présente invention, cette étape est effectuée en utilisant une deuxième résine pour récupérer les polyphénols après leur désorption de la première résine, cette deuxième résine possédant les propriétés suivantes : elle est hydrophobe et non ionique, et permet la désorption des polyphénols par l'utilisation d'un solvant organique.

Selon une mise en oeuvre préférée de l'invention, les étapes suivantes sont réalisées :
(i) la bière partiellement filtrée est mise au contact d'une résine de polyvinylpolypyrrolidone (PVPP) ;
(ii) la PVPP est lavée avec une solution alcaline ;
(iii) la solution de lavage de la PVPP est mise au contact d'une deuxième résine adsorbant les polyphénols, ladite deuxième résine étant hydrophobe et non ionique ;
(iv) les polyphénols sont désorbés de la deuxième résine ;
(v) les polyphénols sont concentrés.

Certaines mises en oeuvre préférées de ces différentes étapes sont détaillées ci-dessous. Ces mises en oeuvres particulières peuvent être utilisées indépendamment ou en combinaison, dans des procédés en continu ou en discontinu (*"batch"*)*.*

A l'étape (ii), la PVPP peut subir deux lavages à la soude ; dans ce cas, seule la solution de soude du premier lavage ou, le cas échéant, une fraction de cette solution, est utilisée à l'étape (iii). De préférence, la concentration de soude est comprise entre 1 et 2 % ; de manière encore préférée, elle est d'environ 1,6%. Bien entendu, une autre solution alcaline peut être utilisée à la place de la soude.

Après désorption des polyphénols par la solution alcaline, cette solution sera de préférence rapidement neutralisée, voire acidifiée, afin d'éviter la dégradation des polyphénols. Ceci est particulièrement avantageux dans le cas d'un procédé discontinu. En effet, dans le cas d'un procédé continu, le temps pendant lequel les polyphénols restent dans la solution alcaline peut être réduit, de façon à s'affranchir du problème de dégradation des polyphénols en milieu alcalin et donc, éventuellement, de l'étape de neutralisation ou acidification. Comme cela est décrit dans la partie expérimentale ci-dessous, l'acide phosphorique est particulièrement bien adapté pour neutraliser ou acidifier la solution alcaline à l'issue de l'étape (ii).

La deuxième résine, utilisée à l'étape (iii), sera avantageusement une résine polymérique adsorbante, hydrophobe et non ionique, de préférence aromatique. A titre d'exemples non limitatifs de telles résines, on peut citer la résine Amberlite™ XAD 1180 de la société Rohm and Haas S.A.S., ainsi que sa version alimentaire (Amberlite™ FPX 68), qui est une résine adsorbante macroréticulée conçue pour l'industrie des boissons, spécialement pour extraire les flavonoïdes. La résine Amberlite™ FPX 66, qui permet de récupérer des molécules plus petites par rapport à FPX 68, peut également être utilisée.

Une étape de rinçage de la deuxième résine, avec une solution aqueuse, sera préférentiellement ajoutée entre les étapes (iii) et (iv).

De manière préférée, la désorption à l'étape (iv) est effectuée en passant une solution d'alcool ou un autre solvant organique. Les polyphénols peuvent ensuite être concentrés, par exemple, par évaporation du solvant organique. La solution concentrée peut être lyophilisée ou séchée, pour obtenir une poudre et améliorer la stabilité du concentré de polyphénols.

Actuellement, la PVPP est la seule résine capable d'adsorber les polyphénols et inscrite sur la liste des auxiliaires technologiques autorisés dans le traitement de la bière en Europe. Toutefois, cette liste est susceptible d'être modifiée. Une variante des procédés décrits ci-dessus est donc envisagée conformément à l'invention, dans laquelle la bière partiellement filtrée est directement mise au contact d'une résine adsorbant les polyphénols et permettant leur désorption par un solvant organique. Pour cela, une résine adsorbante, hydrophobe et non ionique, et de préférence aromatique, telle que les résines XAD 1180, FPX 68 et FPX 66 mentionnées plus haut, peut être avantageusement utilisée. Cette variante permet de s'affranchir des étapes de régénération à la soude et de neutralisation à l'acide, diminue la quantité de sels et permet d'améliorer le rendement d'extraction et la pureté des polyphénols.

Un autre aspect de la présente invention est un concentré de polyphénols susceptible d'être obtenu par un procédé tel que décrit ci-dessus. Les différentes étapes du procédé de brassage subies par ces polyphénols leur confèrent des propriétés originales, et notamment une composition riche en catéchines, épi-catéchines et leurs polymères. Un concentré selon l'invention peut être présenté sous forme solide, par exemple en poudre, ou sous forme liquide, par exemple en solution aqueuse, alcoolique ou dans du glycérol.

Un concentré de polyphénols selon l'invention peut être utilisé pour la préparation d'une composition anti-oxydante, quelle que soit la destination de cette composition.

En particulier, les propriétés anti-oxydantes des concentrés de l'invention en font d'excellents candidats comme composants de produits cosmétiques. Ces concentrés peuvent en effet être ajoutés dans une grande diversité de produits cosmétiques, tels que des crèmes ou des lotions hydratantes pour la peau, des solutions de lavage ou de rinçage de la peau ou des cheveux, des masques, des produits de maquillage, *etc.* Les concentrés de l'invention peuvent être intégrés dans des produits cosmétiques à titre de produits actifs, par exemple pour leurs propriétés anti-oxydantes, hydratantes ou stimulantes, mais également comme agents facilitant la conservation des produits cosmétiques. Un produit cosmétique selon l'invention peut bien entendu être formulé pour une application topique, mais il peut également être formulé pour une application orale, sous forme de gélules, de pastilles, de sirop ou toute autre forme. Les inventeurs ont en effet mis en évidence, sur un modèle de peau en culture, un effet bénéfique des polyphénols au contact des cellules épidermiques, sur la qualité de la peau. Les produits cosmétiques de l'invention peuvent en particulier être utilisés pour hydrater la peau et/ou prévenir ou ralentir son vieillissement. A titre indicatif, des compositions cosmétiques comprenant entre 0,25% et 0,5% d'extrait solide riche en polyphénols (comprenant environ 80% de polyphénols) peuvent être avantgeusement utilisées dans ces indications.

Selon un autre aspect particulier de l'invention, les polyphénols issus du procédé de brassage sont utilisés dans une composition nutraceutique telle qu'un complément alimentaire. Un aliment fonctionnel comprenant des polyphénols susceptibles d'être obtenus selon l'invention est également décrit. Par "aliment fonctionnel", on entend une préparation alimentaire à consommer comme un aliment normal bien qu'elle procure des bénéfices supérieurs à la nutrition classique. A titre d'exemples non limitatifs d'aliments fonctionnels, on peut citer les jus de fruits, sodas et autres boissons sans alcool, les produits à base de céréales, les yaourts et préparations lactées, les barres chocolatées, la margarine et autres matières grasses à tartiner, les biscuits, *etc.* Outre leurs propriétés bénéfiques sur la santé, les polyphénols de l'invention peuvent être utilisés dans des aliments à titre d'agents de conservation, par exemple pour remplacer certains produits de synthèse tels que la vitamine C, le BHA (ButhylHydroxyAnisol) et le BHT (ButylHydroxyToluène).

Des compléments alimentaires comportant un concentré de polyphénols obtenu selon l'invention font également partie de la présente invention. Ces compléments peuvent se présenter, par exemple, sous forme de gélules, poudres ou comprimés. Bien entendu, ils peuvent contenir d'autres principes actifs, tels que des vitamines anti-oxidantes (C et E notamment), des vitamines du groupe B, de la vitamine D, du calcium, du magnésium, des acides gras oméga-3, des phospholipides, des extraits végétaux *etc.*

Une utilisation particulière des concentrés de polyphénols de l'invention, illustrée dans les exemples expérimentaux ci-après, est la préparation d'une composition destinée à améliorer les performances cognitives d'un individu. Les "performances cognitives" désignent ici, au sens large, la mémoire, la capacité d'apprentissage, le raisonnement, mais également la capacité de concentration, l'attention, la résistance au stress, la vitesse de réaction *etc.* Une telle composition peut être présentée sous forme de complément alimentaire, mais également sous toute autre forme, notamment galénique.

Selon un autre aspect, la présente invention concerne l'utilisation d'un concentré de polyphénols susceptible d'être obtenu par un procédé décrit plus haut, pour la préparation d'une composition anti-vieillissement. En particulier, une telle composition peut être utilisée pour limiter ou retarder les conséquences du vieillissement cérébral, telles que les pertes de mémoires, les démences *etc.* Une telle composition anti-vieillissement peut être destinée à un usage humain ou vétérinaire. Toutes les formes d'administration décrites ci-dessus peuvent être envisagées pour une telle composition.

A titre indicatif, les polyphénols de l'invention peuvent être administrés à raison de 100 à 400 mg par jour pour un humain adulte.

Outre les dispositions qui précèdent, l'invention comprend encore d'autres dispositions, qui ressortiront des exemples expérimentaux ci-dessous, et des figures annexées.

### LEGENDE DES FIGURES

Figure 1 : Schéma de principe de la filtration/régénération PVPP.
Figure 2 : Polyphénols présents dans la solution de régénération en fonction du pH.
Figure 3 : Evolution des polyphénols totaux au cours du temps.
Figure 4 : Evolution de l'activité antioxydante en fonction de la température.
Figure 5 : Evolution de la teneur en polyphénols lors d'une régénération.
Figure 6 : Schéma du test pilote.
Figure 7 : Teneur en polyphénols lors de 5 régénérations.
Figure 8 : Schéma de principe - remplacement de la PVPP par une autre résine XAD.
Figure 9 : Evolution pondérale des rats pendant la durée de l'expérimentation (Moyenne ± SEM).
Figure 10 : Consommation alimentaire (g/kg/j) (Moyenne ± SEM).
Figure 11 : Consommation hydrique (g/kg/j) (Moyenne ± SEM).
Figure 12 : Nombre total d'appuis sur les deux leviers lors des 10 minutes de la session d'habituation (Moyenne ± ESM).
Figure 13 : Discrimination entre le levier actif et le levier inactif au cours des 10 minutes de la session d'habituation (Moyenne ± ESM).
Figure 14 : Nombre total d'appuis sur les deux leviers au cours des 20 minutes de la session de test (Moyenne ± ESM).
Figure 15 : Discrimination entre le levier actif et le levier inactif lors des 20 minutes de la session de test (Moyenne ± SEM).
Figure 16 : Temps de latence (s) pour atteindre la zone de la plateforme (Moyenne ± SEM).
Figure 17 : observation microscopique des explants non traités, à J0 (A) et à J6 (B).
Figure 18 : Observation microscopique des explants à J6, traités avec la formulation contenant du rétinol (A) ou avec l'excipient (B).
Figure 19 : Observation microscopique à J6, après coloration des GAGs : Témoin (A), Référence (B), traité avec les polyphénols à 0,5% en topique (C), et traités avec les polyphénols à 0,025% dans le milieu.
Figure 20 : Observation microscopique à J6, après immunomarquage de la laminine-5 : Témoin (A), Référence (B), traité avec les polyphénols à 0,5% en topique (C), et traités avec les polyphénols à 0,025% dans le milieu.
Figure 21 : Observation microscopique à J6, après immunomarquage du collagène III : Témoin (A), Référence (B), traité avec les polyphénols à 0,5% en topique (C), et traités avec les polyphénols à 0,025% dans le milieu.
Figure 22 : Observation microscopique à J6, après immunomarquage du collagène IV : Témoin (A), Référence (B), traité avec les polyphénols à 0,5% en topique (C), et traités avec les polyphénols à 0,025% dans le milieu.
Figure 23 : composition des échantillons en flavolols et acides phénoliques (% masse).

### EXEMPLES

### Exemple 1 : Essais au laboratoire

### 1.1. Contexte

Le procédé de filtration de la bière se déroule en deux étapes :
1. Passage sur un filtre Kieselguhr et élimination des particules solides résiduelles (bière "partiellement filtrée"),
2. Diminution d'environ 40 à 50 % de la concentration de polyphénols dans la bière grâce à la PVPP.

La bière filtrée est ensuite stockée puis conditionnée.

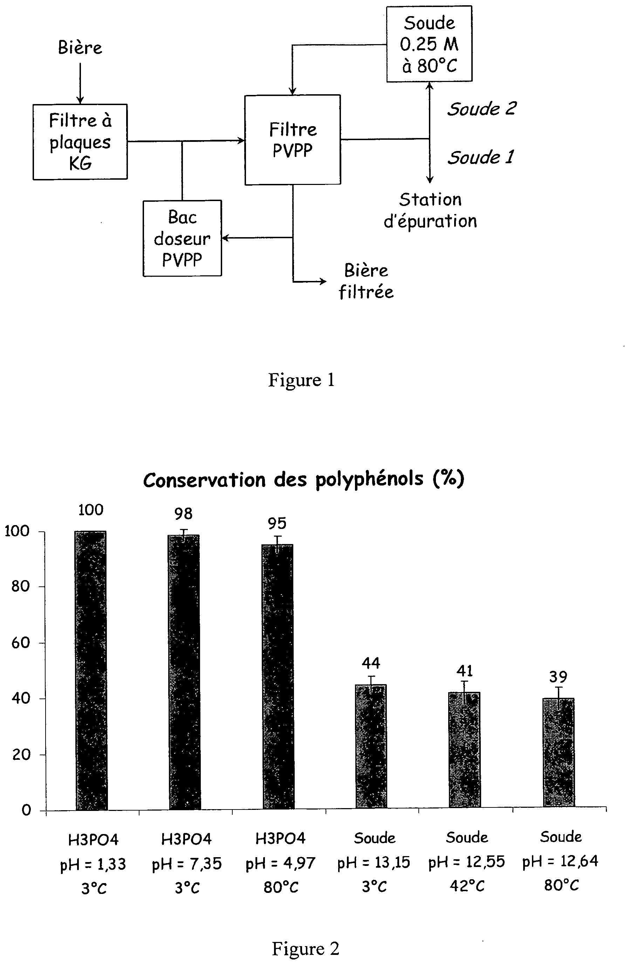

La PVPP est ajoutée à la bière partiellement filtrée en sortie du filtre Kieselguhr. La PVPP adsorbe les polyphénols puis est retenue sur le filtre PVPP. Un lavage du filtre à la soude chaude à 1,6% (2 portions de 120 hL) permet d'éliminer les polyphénols et de régénérer la PVPP pour la prochaine utilisation. La première portion de soude est envoyée à la station d'épuration, la seconde est récupérée et réutilisée lors de la régénération suivante. L'utilisation et la régénération de la PVPP sont représentées figure 1. Dans les exemples qui suivent, la "solution de régénération" désignera la première portion de soude (Soude 1).

### 1.2. Stabilité des polyphénols dans la solution de régénération.

La stabilité des polyphénols de la solution de régénération est déterminée dans différentes conditions (pH, temps, température). Les polyphénols totaux sont dosés à différents temps pour chacune des conditions sur 5 prélèvements réalisés. Deux heures s'écoulent entre le prélèvement et la première mesure.

La figure 2 montre l'influence du pH sur la conservation des polyphénols. En 2 heures, la perte en polyphénols est supérieure à 55 % dans la soude non acidifiée. La solution basique doit être neutralisée ou acidifiée immédiatement à la sortie du filtre PVPP pour être exploitable. L'influence de la température entre 3 et 80 °C est nulle.

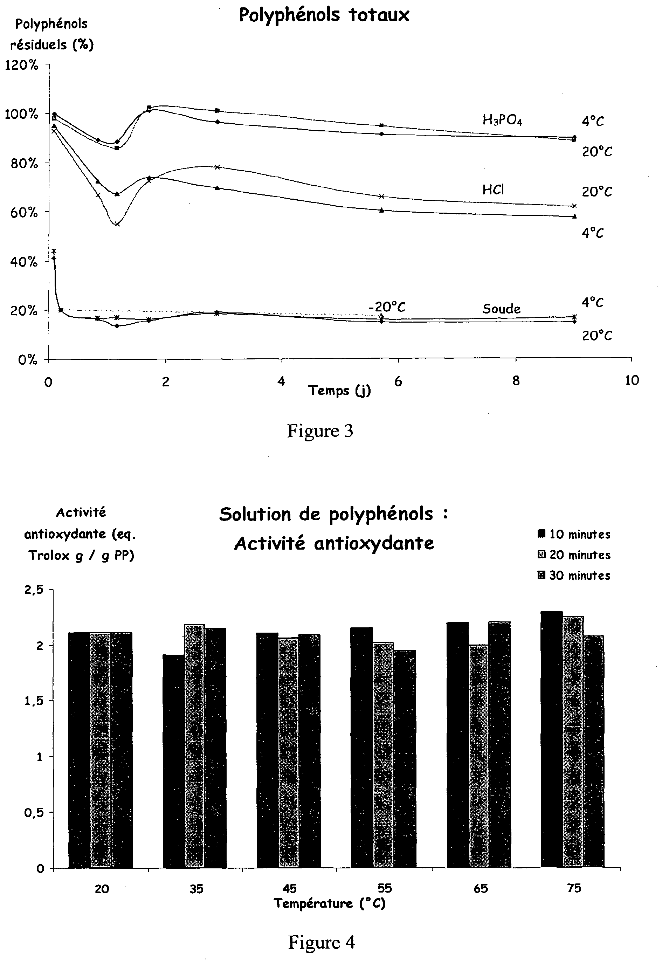

La figure 3 présente l'évolution des polyphénols totaux dans la solution de régénération sur une période de 9 jours. La solution de régénération est prélevée directement en sortie du filtre PVPP. Les échantillons sont conditionnés et immédiatement acidifiés avec de l'acide commercial.

Les conditions suivantes ont été testées :
- Acidité
   - pH compris entre 2 et 3 pour les échantillons acidifiés,
   - pH = 13 pour les échantillons de soude non traités.
- Nature de l'acide utilisé
   - acide phosphorique (H₃PO₄)
   - acide chlorhydrique (HCl)
- Température : -20 °C, 4 °C ou 20 °C

Cette figure montre que :
1. Lorsque la solution est acidifiée à l'acide phosphorique, 88 % des polyphénols sont conservés jusqu'au 9ème jour.
2. Il est préférable d'employer de l'acide phosphorique et non de l'acide chlorhydrique.
3. La solution basique doit être neutralisée immédiatement pour préserver les polyphénols (déjà évoqué plus haut).
4. Quelles que soient les conditions, les polyphénols subissent une transformation. Ce phénomène a été décrit pour les polyphénols du vin (*Tubaro et al.,* 1999). Il s'agirait d'une dimérisation des polyphénols (Brouillard *et al.,* 1997).
5. L'influence de la température (4°C vs. 20 °C) est très faible.

L'influence de la température sur la stabilité des polyphénols a ensuite été mesurée par l'activité antioxydante d'une solution de polyphénols (figure 4). Des échantillons d'une même solution ont été maintenus durant 10, 20 ou 30 minutes à différentes températures (entre 35 et 75 °C). L'activité antioxydante a été mesurée par la méthode DPPH (Brand-Williams *et al.,* 1995).

La figure 4 montre que :
1. L'activité antioxydante (2,11 g eq. Trolox / g polyphénols) est stable entre 20 et 75 °C.
2. Les variations observées (± 5 %) sont liées aux variations expérimentales et ne sont pas significatives.

NB : Ces résultats ont été confirmés sur le lot pilote entre 40 et 70 °C.

### 1.3. Production d'extraits enrichis en polyphénols au laboratoire.

Pour déterminer la composition phénolique de la bière, McMurrough *et al.* ont réalisé une extraction à l'acétate d'éthyle sur de la bière dégazée, puis ont analysé les extrait par HPLC (McMurrough *et al.,* 1984). Une première extraction a été réalisée par cette technique, pour extraire les polyphénols de la solution de régénération.

Les polyphénols ont donc été extraits à partir de la solution de soude de régénération de PVPP en appliquant le protocole suivant :
1. Acidification de la solution de soude jusqu'à pH 2 avec HCl,
2. Ajout d'acétate d'éthyle (ratio soude / acétate d'éthyle : 1 /1,5, v./v.),
3. Agitation 2 heures, vitesse 300-500 rpm puis repos durant 2 heures et séparation des phases,
4. Récupération de la phase organique, évaporation et lyophilisation.

Les caractéristiques des extraits obtenus sont résumées dans le tableau 2 (colonne A)

La méthode précédente permet d'obtenir des polyphénols avec une pureté de 77 %. Le rendement d'extraction est en revanche faible (environ 37 %). D'autres conditions ont été expérimentées afin d'augmenter le rendement d'extraction. Les modifications ont porté sur
- le pH (entre 1 et 7),
- le solvant d'extraction (éther, dichlorométhane),
- l'ajout de chlorure de sodium afin d'améliorer la séparation des phases organique et aqueuse.

Les caractéristiques des extraits obtenus sont résumées dans le tableau 2 (colonne B). Globalement, lors de l'utilisation des ces variantes, le rendement d'extraction est passé à 32 %. Les extraits obtenus contenaient plus de sels que précédemment et le titre en polyphénols n'était que de 42 %. Les différentes variantes n'ont donc pas permis d'améliorer l'efficacité de la technique d'extraction.

Afin de développer un procédé permettant de récupérer les polyphénols en plus grande quantité dans des conditions plus compatibles avec une exploitation industrielle adaptée aux installations existantes et avec les applications envisagées, d'autres techniques de désalage et de concentration des polyphénols ont été envisagées. Ces possibilités sont résumées dans le tableau 1.

**Tableau 1 : Méthodes de concentration et de désalage proposées.**

| Tâche | Méthode | Informations diverses |
|---|---|---|
| Acidification (rapidement, en sortie de filtre PVPP) et désalage | Acidification directe | Génération de sels Nécessité d'élimination des sels |
| | Résine échangeuse de cations | Pas d'étape supplémentaire de désalage |
| | | Régénération de la résine à l'acide (HCl, H₂SO₄) |
| Concentration | Filtration sur membrane | Elimination d'une partie de l'eau, concentration d'un facteur 5 |
| | | Pression 40 - 50 bars |
| | Adsorption polyphénols sur résine | Régénération à l'alcool |
| | | Distillation sous pression réduite |

Le choix s'est porté sur une résine d'adsorption des polyphénols développée par la société Rohm & Haas. Les polyphénols ont donc été extraits de la soude de régénération de PVPP neutralisée en appliquant la méthode suivante :
1. Passage de la solution de soude à travers une colonne contenant la résine XAD1180 et adsorption des polyphénols. La capacité d'adsorption de la résine est de 20 g de polyphénols par litre de résine,
2. Lavage de la résine à l'eau afin d'éliminer les traces de sels et autres impuretés,
3. Elution des polyphénols à l'éthanol,
4. Récupération de la phase organique, évaporation et lyophilisation.

Pour 100 ml de résine, 150 ml d'eau et 150 ml d'alcool ont été utilisés.

Les caractéristiques des extraits obtenus sont résumées dans le tableau 2 (colonne C).

Ce procédé résout les problèmes liés à l'utilisation de l'acétate d'éthyle.
- L'extraction des polyphénols disponibles est augmentée (65 % d'extraction),
- La pureté des extraits passe à 85-90 %,
- L'éthanol est utilisé ou présent dans de nombreux procédés industriels (pharmacie) ou produits de consommation courante (bière, antitussif).

### 1.4. Extraits de polyphénols obtenus au laboratoire.

Les caractéristiques des extraits obtenus au laboratoire par les trois méthodes décrites précédemment sont résumées dans le tableau 2 ci-dessous.

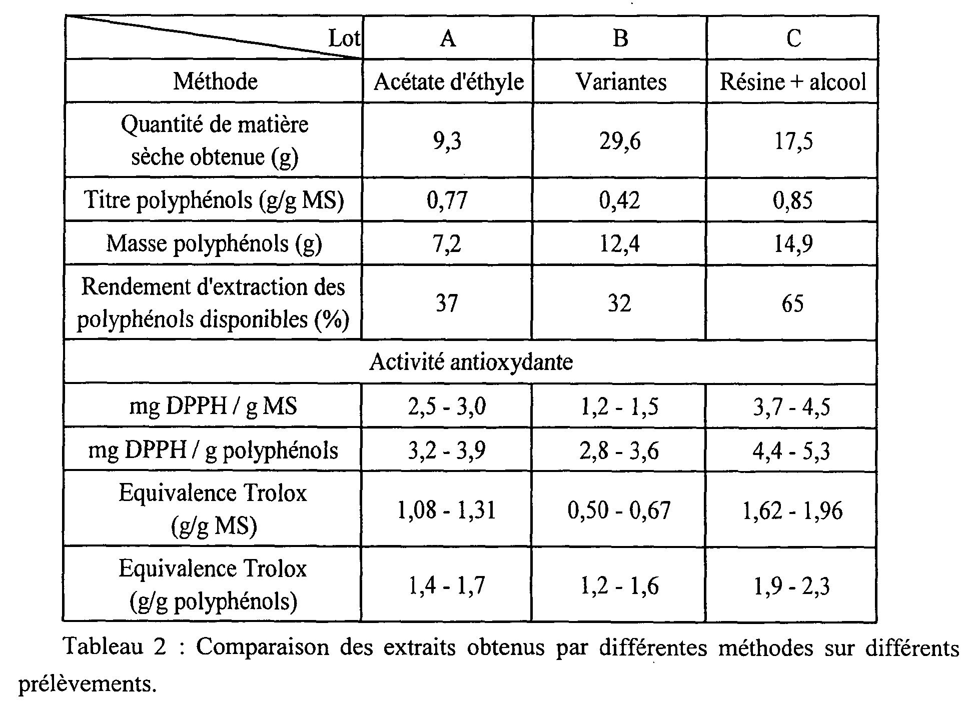

### Exemple 2 : Scale-up, du laboratoire vers la production.

### 2.1. Essai pilote.

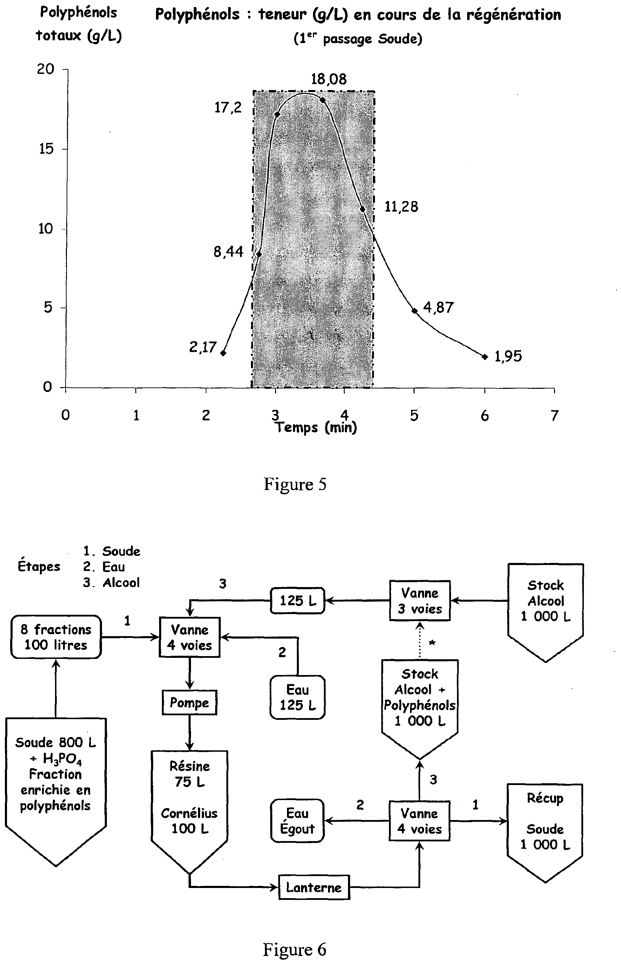

Des prélèvements réalisés ont montré que lors de la régénération de la PVPP, seul le premier lavage à la soude est riche en polyphénols. De plus, la concentration en polyphénols varie au cours de la régénération de la PVPP. Elle est représentée pour une régénération en figure 5.

La quantité de polyphénols est évaluée par intégration de la surface sous la courbe. D'après l'allure de la courbe, environ 65 % des polyphénols contenus dans la solution de régénération sont éliminés durant une période de 2 minutes (partie grisée sur la figure 5). C'est donc durant cet intervalle de temps qu'environ 10 hL de soude ont été prélevés pour la réalisation de l'essai pilote.

La validation de la méthode proposée a nécessité la réalisation d'un essai pilote afin de produire environ 5 à 10 kg de polyphénols.

Le prélèvement de soude a été réalisé directement en sortie du filtre PVPP après suspension de la séquence standard de régénération à la 3ème minute (figure 5, partie grisée). La solution a été immédiatement acidifiée par 20 litres d'acide phosphorique lors du remplissage de la cuve servant au transport.

La teneur en polyphénols de la soude prélevée (800 L) était de 19 g/L. La soude acidifiée (H₃PO₄, pH 5) a été traitée par portions d'environ 100 litres. L'installation utilisée est schématisée sur la figure 6.

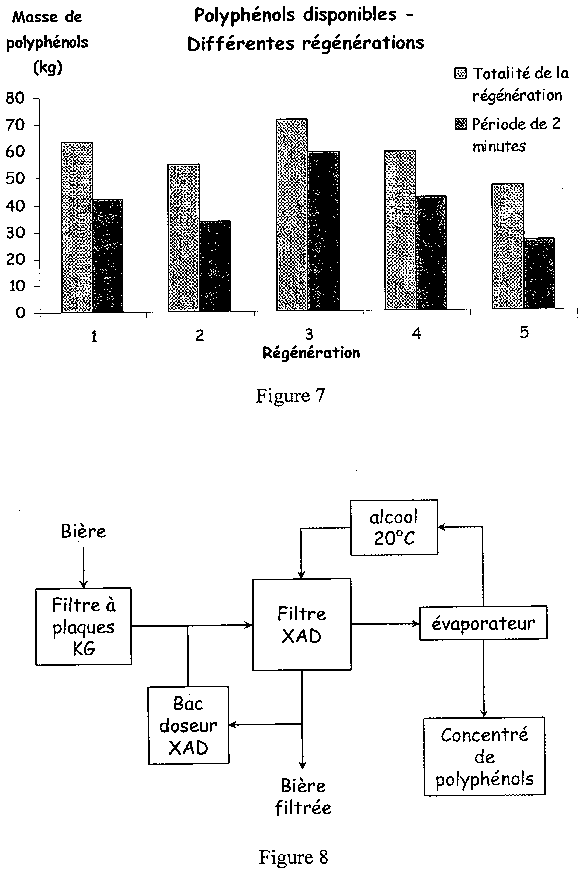

Une variante de cette installation consiste à faire recirculer le solvant organique (flèche en pointillés repérée par une étoile *). Dans ce cas, le volume de solvant organique peut être diminué. Ainsi, par exemple, on peut utiliser 100L d'alcool au lieu de 1000L comme indiqué dans la figure 6. Cette variante présente plusieurs avantages : (i) les polyphénols peuvent être obtenus à des concentrations supérieures ; (ii) l'évaporation devient optionnelle, voire inutile, et on peut se passer d'un évaporateur (ceci est également vrai dans la version où une seule résine est utilisée, comme illustré à la figure 8) ; (iii) la suppression de l'étape d'évaporation, conduisant à une lyophilisation directe du produit, rend le procédé plus économique.

Un filtre métallique a été placé dans le Cornélius la circulation du fluide a été forcée du bas vers le haut, afin d'éviter des problèmes de colmatage.

Le rendement d'extraction, de 60 %, peut être augmenté en améliorant les conditions de traitement de solution de soude.

La quantité de polyphénols isolés était d'environ 9 kg et la pureté supérieure à 80 %. L'activité antioxydante du produit obtenu était plus élevée que lors des essais au laboratoire car la mise en oeuvre du pilote a permis d'éviter les entrées d'air dans le circuit lors du basculement entre les différents liquides et donc la dégradation des polyphénols.

**Tableau 3 : Comparaison des extraits obtenus par la méthode retenue.**

| Lot | Labo | Pilote |
|---|---|---|
| Quantité de Matière Sèche (obtenue au Labo, calculée pour le Pilote) | 17,5 g | 10,96 kg |
| Titre polyphénols (g/g MS) | 0,85 | 0,81 |
| Masse polyphénols | 14,9 g | 8,82 kg |
| Rendement d'extraction des polyphénols disponibles (%) | 65 | 60 |

| Activité antioxydante | | |
|---|---|---|
| mg DPPH / g MS | 3,7 - 4,5 | 5,3 |
| mg DPPH /g polyphénols | 4,4 - 5,3 | 6,6 |
| Equivalence Trolox (g/g MS) | 1,62 - 1,96 | 2,24 |
| Equivalence Trolox (g/g polyphénols) | 1,9 - 2,3 | 2,8 |

### 2.2. Aspects industriels

Afin de vérifier la reproductibilité des résultats obtenus précédemment, ces opérations ont été répétées pour cinq régénérations (figure 7).

En raison de contraintes industrielles, la régénération 5 a été réalisée alors que seulement 75 % de la PVPP disponible étaient utilisés et les quantités de polyphénols déterminées sont plus faibles.

Les prélèvements de soude réalisés sur les différents filtres PVPP présentent des caractéristiques similaires (tableau 4) :
• Le potentiel de polyphénols disponible est d'environ 55 à 65 kg par régénération,
• D'après l'allure de la courbe, 65 % des polyphénols contenus dans la solution de régénération sont concentrés sur une période de 2 minutes (grisée sur la figure 5).

**Tableau 4 : Données caractérisant une régénération.**

| | Passage Soude 1 | Période retenue | Proportion (%) |
|---|---|---|---|
| Durée (minute) | 7 | ∼ 2 | 25 - 30 |
| Volume de soude (hL) | 120 | ∼ 35 | 25 - 30 |
| Polyphénols disponibles (kg) | 55 - 65 | 35 - 45 | 60 - 65 |

### Exemple 3 : Variante de valorisation des polyphénols issus de la bière

Une seconde possibilité de récupération des polyphénols consiste à remplacer la PVPP par la résine développée par Rohm & Haas (figure 8). La désorption de cette résine étant réalisée à l'alcool, les sels sont uniquement apportés par la bière et non par la soude neutralisée à l'acide (consommation nulle de soude et d'acide). Il est possible de proposer un produit titrant minimum 95 % de polyphénols. Outre la pureté, la quantité de polyphénols récupérables augmente pour atteindre 60 kg par régénération. Pour être mise en oeuvre industriellement, cette variante nécessite une modification de la liste des auxiliaires technologiques autorisés dans le traitement de la bière, ainsi qu'un travail de développement pour vérifier l'impact de ces modifications sur les caractéristiques physiques et organoleptiques de la bière.

### Exemple 4 : Etude des effets préventifs des polyphénols issus des co-produits de la filière brassicole sur l'activité antioxydante et sur la sphère cognitive suite à un coup de chaleur chez des rats mâles Sprague Dawley adultes

### 4.1. Introduction

Les effets préventifs de polyphénols issus des co-produits de la filière brassicole, sur l'activité antioxydante, ainsi que sur la sphère cognitive suite à un coup de chaleur, ont été évalués chez des rats mâles Sprague Dawley adultes.

Les animaux des différents groupes ont été traités pendant 3 semaines (J1 à J22) avec les polyphénols administrés par voie orale aux doses de 25 et 50 mg/kg, ou avec le véhicule (eau de source). L'activité antioxydante des polyphénols a été évaluée sur sang total en début et en fin de traitement préventif, puis le lendemain du coup de chaleur réalisé au jour 16. Les tests d'apprentissage ont été réalisés d'une part dans le modèle de conditionnement d'évitement d'un stimulus lumineux aversif (conditionnement opérant) au jour 19 et dans le test du labyrinthe aquatique de Morris (mémoire spatiale) aux jours 21 et 22, pour évaluer les effets protecteurs des polyphénols contre les déficits cognitifs induits par une production excessive de radicaux libres suite au coup de chaleur.

### 4.2. Matériels et méthodes

### Animaux

Soixante-quatre rats mâles Sprague Dawley (Harlan, Hollande) d'un poids de 250 à 275 g ont été utilisés. A la réception, les rats ont été marqués et répartis par groupes de 4 dans des cages en polycarbonate de type F (48 x 27 x 20 cm, U.A.R., 91 - Epinay-Sur-Orge, France). Les animaux ont été stabulés dans une animalerie climatisée à une température de 22 ± 1°C. Les rats ont disposé de nourriture classique 2016 (Harlan, France) et de boisson *ad libitum* et ont été soumis à un cycle lumière-obscurité inversé de 12 heures.

Après une familiarisation d'une semaine aux conditions du laboratoire, les rats ont été pesés et répartis au hasard en 4 groupes de traitements (n = 16 rats/groupe) :
- groupe "TB" : Témoin Blanc traité avec le véhicule et ne subissant pas de coup de chaleur ;
- groupe "TCC" : Témoin Coup de Chaleur traité avec le véhicule et subissant le coup de chaleur ;
- groupe "PP25" : traité à la dose de 25 mg/kg de polyphénols et subissant le coup de chaleur ;
- groupe "PP50" : traité à la dose de 50 mg/kg de polyphénols et subissant le coup de chaleur.

Afin d'éviter les interférences éventuelles entre les divers produits, les rats d'une même cage ont tous reçu le même traitement. Les rats des différents groupes ont été manipulés de la même façon et dans les mêmes conditions.

### Administration des produits et des tests (Tableau 5)

Les polyphénols, solubilisés dans de l'eau de source (5 ml/kg) aux doses de 25 et de 50 mg/kg, et le véhicule ont été administrés par voie intragastrique aux animaux chaque jour pendant 3 semaines. Suite à la délivrance du traitement, les animaux de tous les groupes ont été nourris *ad libitum* avec le régime standard.

Cinq cent microlitres de sang total ont été prélevés sur 12 rats par groupe à J0 et à J15 pour évaluer l'activité antioxydante des polyphénols. Les prélèvements ont été effectués au niveau de la veine caudale à l'aide d'une aiguille, le sang a été directement recueilli dans un tube EDTA. Cette méthode permet de collecter de petits volumes de sang sans sacrifier l'animal. Une habituation au dispositif du test de conditionnement d'évitement d'un stimulus lumineux aversif (TESLA) a été réalisée pendant 10 minutes à J15 afin de comparer l'activité manipulatoire des animaux des 4 groupes. Un coup de chaleur a été réalisé à J16 et un nouveau prélèvement sanguin a été réalisé à J17 pour évaluer les effets protecteurs des polyphénols contre l'excès de production de radicaux libres et vérifier l'activité antioxydante chez les animaux ayant préalablement ingéré le produit. Trois jours après le coup de chaleur (J19), les animaux ont été testés dans le TESLA pour évaluer leurs performances d'apprentissage en fonction des produits ingérés. Aux jours 21 et 22, les rats ont été testés dans le labyrinthe aquatique de Morris pour évaluer leur acquisition d'un apprentissage spatial et leur mémoire à court et à long termes.

Les animaux ont été pesés tous les deux jours afin d'adapter la quantité de polyphénols à leur administrer quotidiennement en fonction de leurs poids corporels.

Les prises alimentaire et hydrique des animaux ont été relevées pendant les deux semaines précédant le coup de chaleur et la semaine suivante.

**Tableau 5 : Protocole d'administration des produits et des tests**

| **Groupes** | n | Traitement (J1 à J22) | Dose mg/kg /j | Coup de chaleur (CC) (n = 16) | Mesure de l'activité antioxydante (n = 12) | Habituation TESLA | Test du TESLA | Test de Morris |
|---|---|---|---|---|---|---|---|---|
| **TB** | 16 | Eau de source | - | Non | Jour 0, J15 et J17 | J15 | J19 | J21 et J22 |
| **TCC** | 16 | Eau de source | - | J16 | | | | |
| **PP25** | 16 | Polyphénols | 25 | J16 | | | | |
| **PP50** | 16 | Polyphénols | 50 | J16 | | | | |

### Procédures expérimentales

### Justification du modèle de coup de chaleur

Le coup de chaleur expérimental reproduit les effets d'une exposition prolongée à la chaleur. Il existe différents modèles animaux de coup de chaleur, le plus répandu étant le modèle murin.

Chez le rat anesthésié exposé à la chaleur, il existe un phénomène ischémique cérébral terminal avec une intense libération de dopamine, de sérotonine, de glutamate et de monoxyde d'azote (Yang et Lin, 2002 ; Canini *et al.,* 2001). Il n'est donc pas surprenant de trouver à ce moment une augmentation des marqueurs de peroxydation membranaire et des lésions cérébrales.

Chez le rat vigile exposé à la chaleur (Tdb = 40°C), il est impossible pour des raisons éthiques d'aller jusqu'au phénomène ischémique terminal. Néanmoins, il est possible de suivre l'évolution triphasique de la température centrale : immédiatement après le début de l'exposition, la température augmente puis se stabilise dans un plateau de thermorégulation qui se termine par une ascension thermique et se poursuit jusqu'au décès de l'animal. L'arrêt de l'exposition à une température corporelle de 41.5 - 42.0°C est alors un bon compromis : les animaux sont dans la dernière phase d'ascension thermique et le taux de survie est important (de l'ordre de 80%).

Dans ce cadre, des altérations comportementales de type hyperactivité manipulatoire et déficit cognitif ont été évalués quelques jours après l'exposition au coup de chaleur dans le test de conditionnement d'évitement d'un stimulus lumineux aversif et dans le test du labyrinthe aquatique de Morris.

### Coup de chaleur

Après avoir pris la température rectale initiale du rat, l'animal a été placé à 40°C dans le dispositif de coup de chaleur. Lorsque la température corporelle du rat a atteint 41.5°C, il a été retiré du dispositif et remis dans sa cage.

### Activité antioxydante

L'activité antioxydante a été mesurée à l'aide du test KRL (Kirial International, France) (Prost *et al.,* 1992). Il consiste à soumettre un échantillon de sang à une agression radicalaire dans des conditions contrôlées et standardisées. Tous les systèmes enzymatiques et chimiques de l'échantillon se mobilisent pour protéger l'intégrité des cellules jusqu'à leur lyse. La mesure de la diminution de l'absorbance permet de suivre la disparition progressive des cellules. La résistance du sang à l'attaque radicalaire est exprimée par le temps nécessaire à la lyse de 50% des cellules sanguines (Temps de demi-hémolyse).

Le test a été réalisé à l'aide du Kit KRL (Réf KRLSPI101/103/105) dans des microplaques de 96 puits et les résultats ont été analysés avec le logiciel KRL (version 3.02).

L'analyse du sang total permet de mesurer les défenses intracellulaires et extracellulaires, ce qui donne une indication instantanée de l'état physiologique du rat au moment du prélèvement de sang.

### Test de conditionnement d'évitement d'un stimulus lumineux aversif (TESLA)

Ce modèle utilise l'aversion du rat pour un environnement fortement éclairé. Dans un premier temps, le rat apprend à maîtriser son environnement lumineux aversif dans le cadre d'un conditionnement d'évitement : l'animal apprend à appuyer sur un levier actif pour obtenir des périodes d'obscurité comme renforcement positif (Messaoudi *et al.,* 1996 ; Messaoudi *et al.,* 1999).

Le dispositif expérimental consiste en une cage isolée (50 x 40 x 37 cm), fortement éclairée (1200 lux) et comportant deux leviers : l'un actif, permettant d'obtenir 30 secondes d'obscurité comme renforcement positif lorsqu'il est actionné et l'autre est inactif. Les appuis sur le levier actif pendant la période d'obscurité ne procurent pas de périodes d'obscurité supplémentaires.

La batterie de test, composée de 4 dispositifs de conditionnement, est entièrement automatisée et pilotée par ordinateur. L'habituation à ce dispositif a été réalisée la veille du coup de chaleur à J15. Le jour du test (J19), la procédure d'acquisition de l'apprentissage discriminant a été réalisée sur une période de 20 minutes, 3 jours après le coup de chaleur.

Les variables enregistrées étaient les nombres d'appuis sur les leviers actif (LA) et inactif (LI) ainsi que le nombre de périodes d'obscurité obtenues.

Les nombres d'appuis actifs et inactifs ont permis d'évaluer le niveau de l'activité manipulatoire. L'acquisition de l'apprentissage (discrimination entre les deux leviers) a été évaluée en comparant les nombres cumulés d'appuis sur chacun des deux leviers (LA vs. LI).

### Test du labyrinthe aquatique de Morris : mémoire spatiale

Le rat, déposé dans un bassin circulaire (0150 cm) rempli d'eau, nage et cherche à fuir le milieu aquatique aversif en se réfugiant sur une plate-forme immergée à 2 mm sous la surface de l'eau. La session de test comporte 5 essais successifs au cours desquels l'animal apprend à situer l'emplacement de la plate-forme immergée et à s'y réfugier. Un temps de repos de 30 secondes sur la plate-forme est observé entre deux essais pour permettre au rat de prendre ses repères spatiaux, indispensables à son orientation dans le dispositif.

Le lendemain, le rat est à nouveau placé dans les mêmes conditions expérimentales et un essai unique est réalisé (*retest*).

Ce test a permis, au jour 21, d'évaluer les performances exploratoires et cognitives dans la situation d'apprentissage spatial progressif (mémoire à court terme), et lors du retest (J22), d'évaluer la mémoire à long terme du rat.

La variable étudiée est le temps de latence pour atteindre l'emplacement de la plate-forme immergée Blokland *et al.,* 2004 ; Morris *et al.,* 1982).

### Analyse statistique

En fonction de la distribution gaussienne ou non des données, des tests statistiques paramétriques (P) ou non-paramétriques (NP) ont été utilisés : l'analyse de la variance (ANOVA) en mesures factorielles (P) ou test de Kruskal-Wallis (NP) suivis le cas échéant par le test t non apparié (P) ou le test de Mann-Whitney (NP) pour comparer les groupes traités au groupe témoin. Pour l'analyse des mesures répétées ou appariées, l'ANOVA en mesures répétées (P) ou le test de Friedman (NP) ont été utilisés, suivis le cas échéant par des tests de comparaisons deux à deux : test t apparié (P) ou test de Wilcoxon (NP).

Les traitements statistiques ont été réalisés à l'aide du logiciel Statview 5 (SAS, Institute Inc.).

### 4.3. Résultats

Remarque : Au cours de cette étude, trois rats sont morts avant la fin de l'expérimentation.

### Evolution pondérale

Du jour 0 au jour 16, l'analyse de la variance n'a pas montré d'hétérogénéité significative parmi les poids des rats des différents groupes.

Du jour 17 au jour 22, l'analyse de la variance a montré une hétérogénéité significative parmi les poids des rats des différents groupes (Tableau 6).

Le test t non apparié a montré qu'aux jours 17 et 22, les poids des rats du groupe TCC ont été significativement inférieurs à ceux des rats du groupe TB. Aux jours 18, 20 et 21, les poids des rats du groupe TCC ont montré une tendance à être inférieurs à ceux des rats du groupe TB.

Du jour 17 au jour 22, les poids des rats des groupes PP25 et PP50 ont été significativement inférieurs à ceux des rats du groupe TB.

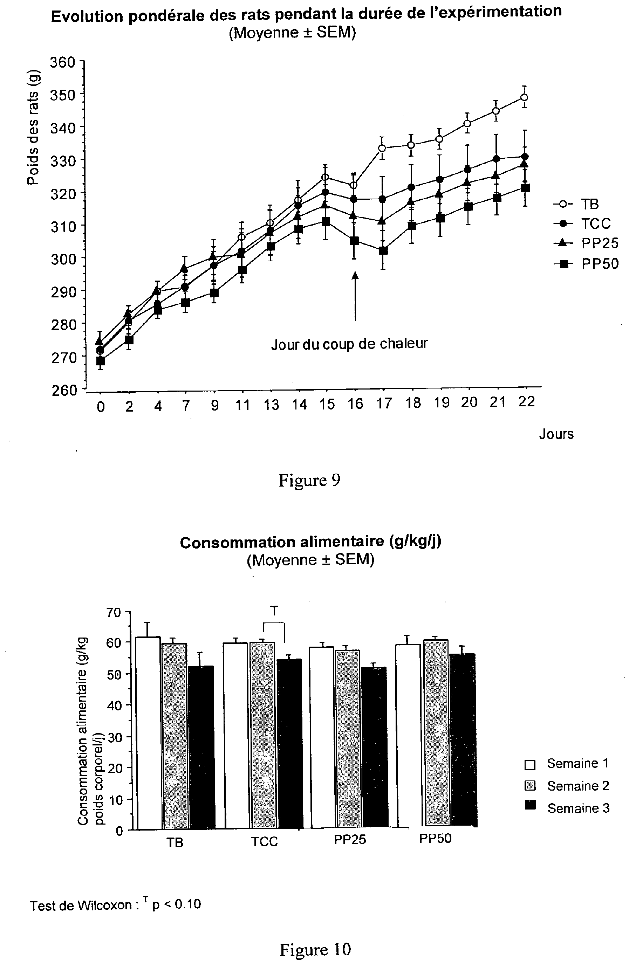

Les poids des rats des groupes PP25 et PP50 n'ont pas été significativement différents de ceux des rats du groupe TCC, à l'exception du jour 17 où les poids des rats du groupe PP50 ont montré une tendance à être inférieurs à ceux des rats du groupe TCC (Tableau 7 ; Figure 9).

**Tableau 6 : Analyse de la variance des poids des rats des quatre groupes de traitement.**

| **Jours** | **0** | **2** | **4** | **7** | **9** | **11** | **13** | **14** | **15** | **16** | **17** | **18** | **19** | **20** | **21** | **22** |
|---|---|---|---|---|---|---|---|---|---|---|---|---|---|---|---|---|
| **ANOVA: F_{(3,57)} Signifcativité** | 0.74 N.S. | 1.05 N.S. | 0.95 N.S. | 1.27 N.S. | 0.93 N.S. | 0.50 N.S. | 0.27 N.S. | 0.47 N.S. | 0.93 N.S. | 1.57 N.S. | 5.63 P< 0.01 | 3.76 P< 0.05 | 3.38 P< 0.05 | 3.94 P< 0.05 | 4.12 P< 0.05 | 4.41 P< 0.01 |

**Tableau 7 : Comparaison transversale des poids des rats des différents groupes (test t non apparié).**

| **Jours** | **17** | **18** | **19** | **20** | **21** | **22** |
|---|---|---|---|---|---|---|
| **TCC *vs*. TB** | t = 2.07 | t = 1.73 | t = 1.57 | t = 1.82 | t = 1.80 | t = 2.14 |
| | P < 0.05 | P < 0.10 | N.S. | P < 0.10 | P < 0.10 | P < 0.05 |
| **PP25 *vs*. TB** | t = 3.32 | t = 2.70 | t = 2.76 | t = 3.04 | t = 3.28 | t = 3.45 |
| | P < 0.05 | P < 0.05 | P < 0.01 | P < 0.01 | P < 0.01 | P < 0.01 |
| **PP50 *vs*. TB** | t = 4.61 | t = 3.94 | t = 3.79 | t = 4.03 | t = 4.20 | t = 4.32 |
| | P < 0.0001 | P < 0.001 | P < 0.001 | P < 0.001 | P < 0.001 | P < 0.001 |
| **PP25 *vs.* TCC** | t = 0.77 | t = 0.52 | t = 0.51 | t = 0.47 | t = 0.54 | t = 0.26 |
| | N.S. | N.S. | N.S. | N.S. | N.S. | N.S. |
| **PP50 *vs*. TCC** | t = 1.74 | t = 1.35 | t = 1.26 | t = 1.24 | t = 1.22 | t = 0.99 |
| | P < 0.10 | N.S. | N.S. | N.S. | N.S. | N.S. |

Interprétation : après 15 jours de traitement avec les polyphénols aux doses de 25 et 50 mg/kg, l'évolution pondérale des groupes traités aux polyphénols a suivi celle des rats du groupe témoin. Aucun effet toxique des polyphénols n'a été constaté.

Le coup de chaleur a entraîné une perte de poids significative des groupes TCC, PP25 et PP50 par rapport au témoin TB. Il n'y a pas eu de différence significative entre le groupe TCC et les groupes PP25 et PP50 après le coup de chaleur, les évolutions pondérales des rats de ces groupes ont été similaires.

L'ANOVA en mesures répétées appliquée à l'évolution pondérale des rats de chacun des quatre groupes entre J17 et J20, a montré une hétérogénéité significative après le coup de chaleur (Tableau 8).

**Tableau 8 : Comparaison des poids des rats des différents groupes entre J17 et J20.**

| **Groupes** | **TB (n = 16)** | **TCC (n = 15)** | **PP25 (n = 15)** | **PP50 (n = 15)** |
|---|---|---|---|---|
| **ANOVA en mesures répétées de J17 à J20** | F = 20.22 | F = 6.21 | F = 47.68 | F = 4.83 |
| | P < 0.0001 | P < 0.01 | P < 0.0001 | P < 0.01 |

La comparaison entre J17 et J18 par le test t apparié a montré une augmentation significative des poids des rats des groupes PP25 (t = 5.97, p < 0.0001) et PP50 (t = 7.62, p < 0.0001). Aucune différence significative n'a été observée pour les poids des rats des groupes TB (t = 0.77, p = 0.45) et TCC (t = 1.26, p = 0.23).

La comparaison entre J18 et J19 a montré une augmentation significative des poids des rats des groupes PP25 (t = 3.06, p = 0.008) et PP50 (t = 2.97, p = 0.010) et une tendance à l'augmentation du poids des rats du groupe TB (t = 1.93, p = 0.073). Aucune différence significative n'a été observée pour le poids des rats du groupe TCC (t = 0.94, p = 0.365).

La comparaison entre J19 et J20 a montré une augmentation significative des poids des rats des groupes TB (t = 6.48, p < 0.0001), TCC (t = 4.69, p = 0.0003) et PP25 (t = 4.75, p = 0.0003). Aucune différence significative n'a été observée pour le poids des rats du groupe PP50 (t = 0.68, p = 0.507).

Interprétation : Les rats TB ont suivi une courbe de prise de poids classique après le jeûne du jour du coup de chaleur, tandis que les rats traités avec les polyphénols ont repris du poids dès le lendemain du coup de chaleur et ce pendant 3 jours consécutifs pour le groupe PP25 et 2 jours pour le groupe PP50 ; les rats du groupe TCC n'ont commencé à reprendre du poids que le troisième jour après le coup de chaleur (J19).

### Prise alimentaire

Le coup de chaleur a été administré aux animaux en début de semaine 3.

Le test de Kruskal-Wallis n'a pas montré d'hétérogénéité significative pour les consommations alimentaires des rats des groupes TB, TCC, PP25 et PP50 au cours des semaines 1, 2 et 3.

L'analyse des mesures répétées à l'aide du test de Friedman a montré une hétérogénéité significative pour la consommation alimentaire au cours des semaines 1, 2 et 3 pour les rats du groupe TCC (χ² = 6 ; ddl = 2 ; p = 0.05). La consommation alimentaire des rats du groupe TCC a montré une tendance à diminuer entre la semaine 2 et la semaine 3 (test de Wilcoxon : z = 1.826 ; p = 0.068) (Tableau 9 ; Figure 10).

**Tableau 9 : Comparaison de la consommation alimentaire des rats des différents groupes (g/kg/j) ; (Moyenne ± SEM).**

| | **TB (n = 4)** | **TCC (n = 4)** | **PP25 (n = 4)** | **PP50 (n = 4)** | **Analyse non-appariée Kruskal-Wallis** |
|---|---|---|---|---|---|
| **Semaine 1** | 61.80 ± 4.58 | 59.33 ± 1.28 | 58.15 ± 0.88 | 58.40 ± 3.19 | H = 1.853 |
| | | | | | ddl = 3 ; N.S. |
| **Semaine 2** | 59.36 ± 1.97 | 59.93 ± 0.49 | 56.82 ± 1.34 | 59.25 ± 0.91 | H = 2.934 |
| | | | | | ddl = 3 ; N.S. |
| **Semaine 3** | 52.29 ± 4.13 | 54.08 ± 1.49 | 50.93 ± 1.75 | 55.10 ± 2.33 | H = 1.588 |
| | | | | | ddl = 3 ; N.S. |
| | | | | | |
| **Test de Friedman** | χ² = 4.50 | χ² = 6.00 | χ² = 4.50 | χ² = 3.50 | - |
| | ddl = 2 | ddl = 2 | ddl = 2 | ddl = 2 | |
| | N.S. | p < 0.05 | N.S. | N.S. | |
| **Test de Wilcoxon Sem 1 *vs.* Sem 2** | - | z = 0 | - | - | - |
| | | N.S. | | | |
| **Sem 2 *vs.* Sem 3** | - | z = 1.826 | - | - | - |
| | | p < 0.10 | | | |

Interprétation : Les rats du groupe TCC ont montré une tendance à consommer moins d'aliment après le coup de chaleur que pendant les deux premières semaines de l'étude.

### Consommation hydrique

Le test de Kruskal-Wallis n'a pas montré d'hétérogénéité significative pour les consommations hydriques des différents groupes au cours des semaines 1, 2 et 3.

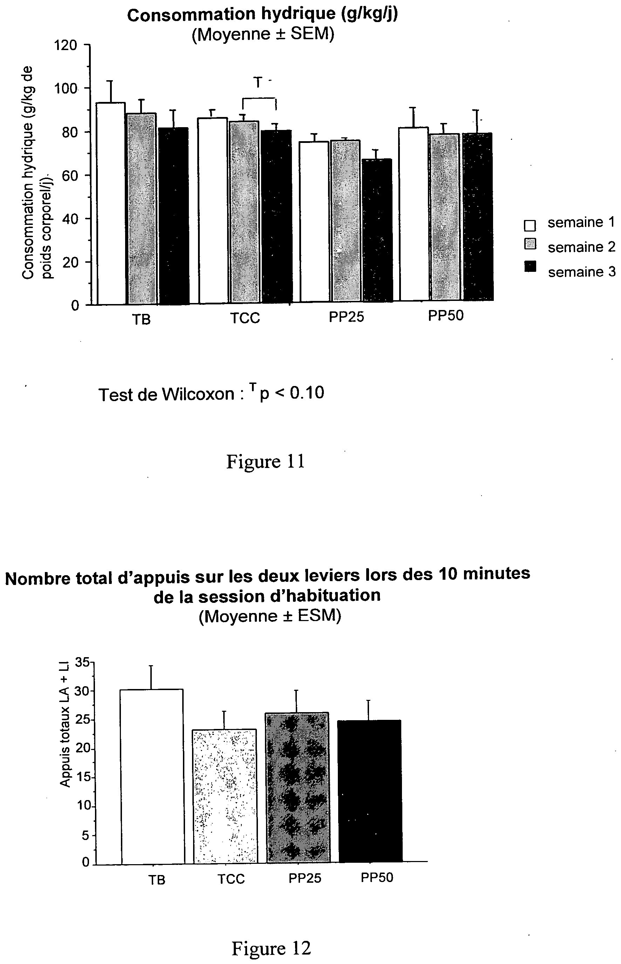

Le test de Friedman a mis en évidence une différence significative pour la consommation hydrique au cours des semaines 1, 2 et 3 pour les rats du groupe TCC (χ² = 6.50 ; ddl = 2 ; p = 0.039). La consommation hydrique des rats du groupe TCC a montré une tendance à diminuer entre la semaine 2 et la semaine 3 (test de Wilcoxon : z = 1.83 ; p = 0.068) (Tableau10, Figure 11).

**Tableau 10 : Consommation hydrique des rats des différents groupes (g/kg/j) (Moyenne ± SEM).**

| | **TB (n = 4)** | **TCC (n = 4)** | **PP25 (n = 4)** | **PP50 (n = 4)** | **Analyse non- appariée Kruskal-Wallis** |
|---|---|---|---|---|---|
| **Semaine 1** | 92.91 ± 10.02 | 85.91 ± 2.14 | 73.66 ± 3.09 | 79.96 ± 9.10 | H = 4.301 |
| | | | | | ddl = 3 ; N.S. |
| **Semaine 2** | 88.10 ± 6.45 | 84.03 ± 2.94 | 74.16 ± 1.20 | 77.03 ± 4.28 | H = 4.985 |
| | | | | | ddl = 3 ; N.S. |
| **Semaine 3** | 81.44 ± 7.80 | 79.09± 3.57 | 65.70 ± 4.56 | 79.91 ± 10.41 | H = 3.728 |
| | | | | | ddl = 3 ; N.S. |
| | | | | | |
| **Test de Friedman** | χ² = 4.50 | χ²=6.50 | χ² = 4.50 | χ² = 0.50 | - |
| | ddl = 2 | ddl = 2 | ddl = 2 | ddl = 2 | |
| | N.S. | p < 0.05 | N.S. | N.S. | |
| **Test de Wilcoxon Sem 1 *vs* Sem 2** | - | z = 0.365 N.S. | - | - | - |
| **Sem 2 *vs* Sem 3** | - | z = 1.826 p < 0.10 | - | - | - |

Interprétation : Les rats du groupe TCC ont eu tendance à consommer moins d'eau après le coup de chaleur que pendant les deux premières semaines de l'étude.

### Activité antioxydante

Au jour 17, l'ANOVA en mesures factorielles a montré une tendance à l'hétérogénéité entre les temps de demi-hémolyse des rats des quatre groupes.

Le test t non apparié n'a pas montré de différence significative entre les groupes.

L'ANOVA en mesures répétées a montré une tendance à la différence entre les temps de demi-hémolyse des trois prélèvements des rats du groupe TB. Le test t apparié n'a pas montré de différence significative entre les différents prélèvements (Tableau 11).

**Tableau 11 : Temps de demi-hémolyse du sang total (min) entre les trois prélèvements (Moyenne ± SEM).**

| | **TB (n = 12)** | **TCC (n = 11)** | **PP25 (n = 11)** | **PP50 (n = 11)** | **ANOVA en mesures factorielles** |
|---|---|---|---|---|---|
| **J0** | 67.28 ± 0.85 | 68.14 ± 1.08 | 70.86 ± 1.56 | 68.48 ± 0.93 | F(3,41) = 1.86 |
| | | | | | N.S. |
| **J15** | 69.84 ± 1.18 | 69.86 ± 1.04 | 70.44 ± 1.41 | 70.05 ± 1.14 | F(3,41) = 0.05 |
| | | | | | N.S. |
| **J17** | 65.23 ± 1.72 | 71.27 ± 2.22 | 69.44 ± 1.01 | 70.49 ± 1.56 | F(3,41) = 2.64 |
| | | | | | p < 0.10 |
| **ANOVA en mesures répétées** | F_{(2,22}) = 2.71 | F_{(2,20)} = 1.37 | F_{(2,20)} = 1.07 | F_{(2,20)} = 2.03 | - |
| | p < 0.10 | N.S. | N.S. | N.S. | |

Interprétation :: Ces trois prélèvements n'ont pas permis de mettre en évidence une modification de l'activité antioxydante du sang total entre les groupes de rats. Un dosage sur hématies, en complément du dosage sur sang total, aurait permis de déduire l'activité antioxydante du plasma et d'obtenir une information plus précise de l'activité antioxydante circulante.

Entre les prélèvements J0 et J17, le test t apparié a montré une augmentation significative du temps de demi-hémolyse des rats du groupe PP50 (t = 2.57, p = 0.026) (Tableau 12).

**Tableau 12 : Temps de demi-hémolyse du sang total (min) entre les prélèvements à J0 et J17 (Moyenne ± SEM).**

| | **TB (n = 12)** | **TCC (n = 11)** | **PP25 (n = 11)** | **PP50 (n = 12)** | **ANOVA en mesures factorielles** |
|---|---|---|---|---|---|
| **J0** | 67.28 ± 0.85 | 68.14 ± 1.08 | 70.86 ± 1.56 | 68.26 ± 0.88 | F_{(3,41)} = 1.92 |
| | | | | | N.S. |
| **J17** | 65.23 ± 1.72 | 71.27 ± 2.22 | 69.44 ± 1.01 | 70.75 ± 1.45 | F_{(3,41)} = 2.81 |
| | | | | | p < 0.10 |
| **Test t apparié** | t= 1.05 | t = 1.52 | t = 1.36 | t = 2.57 | - |
| | N.S. | N.S. | N.S. | p < 0.05 | |

Interprétation : Le traitement à 50 mg/kg de polyphénols permet d'augmenter le temps de demi-hémolyse du sang total entre J0 et J17, conséquence d'une activité antioxydante plus efficace. Dans ces conditions de traitement (dose, durée) et de dosage (sur sang total), seul un effet des polyphénols administrés à 50 mg/kg a été détecté par cette méthode.

### Habituation au test d'évitement d'un stimulus lumineux aversif

Remarque : l'habituation au TESLA a été réalisée à J15, la veille du coup de chaleur.

### Activité manipulatoire des leviers

A l'issue des 10 minutes d'habituation au test, l'ANOVA en mesures factorielles a montré que le nombre d'appuis totaux sur les deux leviers des rats des différents groupes ont été statistiquement équivalents (Tableau 13 ; Figure 12).

**Tableau 13 : Nombre total d'appuis sur les deux leviers au cours de l'habituation (Moyenne ± SEM).**

| | **TB (n = 16)** | **TCC (n = 16)** | **PP25 (n = 16)** | **PP50 (n = 15)** |
|---|---|---|---|---|
| **LA + LI** | 30.06 ± 4.20 | 23.06 ± 3.14 | 25.81 + 3.92 | 24.40 ± 3.38 |
| **ANOVA en mesures factorielles** | F_{(3,59)} = 0.68 | | | |
| | N.S. | | | |

Interprétation : Avant le coup de chaleur, les rats des différents groupes ont montré la même activité manipulatoire.

### Discrimination entre les deux leviers

Pour évaluer la discrimination entre les leviers actifs et inactifs, seuls les appuis pendant la phase lumineuse ont été pris en compte. Seuls les rats ayant appuyé au moins une fois sur chaque levier ont été pris en compte dans l'analyse statistique.

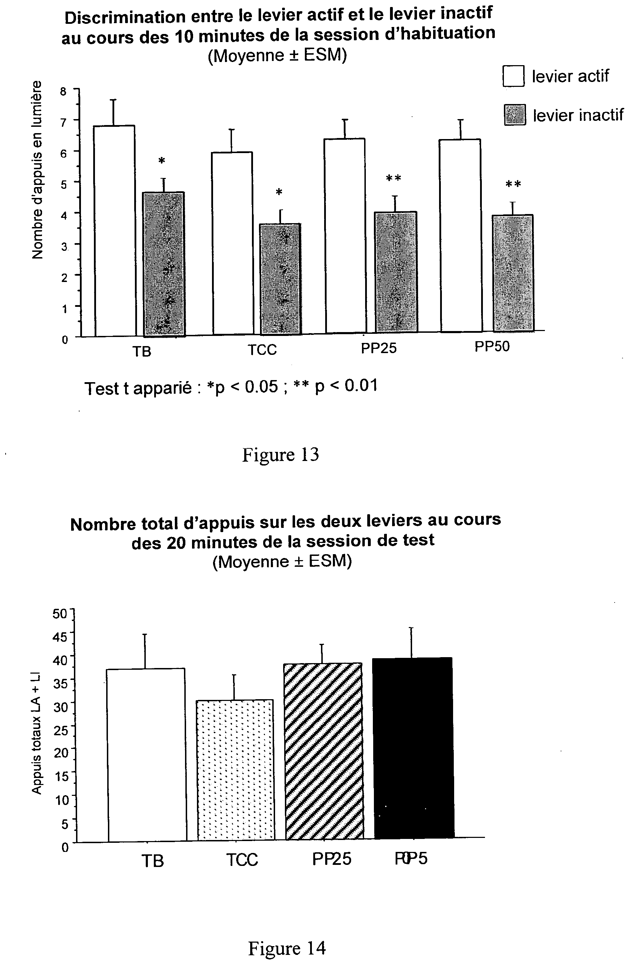

Pendant les 10 minutes d'habituation, le test t apparié a montré que les rats des quatre groupes de traitements ont discriminé significativement le levier actif du levier inactif (Tableau 14 ; Figure 13).

**Tableau 14 : Discrimination entre les leviers actif et inactif au cours de l'habituation (Moyenne ± SEM).**

| **Groupes** | **TB (n = 16)** | **TCC (n = 16)** | **PP25 (n = 16)** | **PP50 (n = 15)** |
|---|---|---|---|---|
| **LA** | 6.63 ± 0.79 | 5.76 ± 0.70 | 6.25 ± 0.57 | 6.20 ± 0.61 |
| **LI** | 4.63 ± 0.46 | 3.56 ± 0.51 | 3.88 ± 0.55 | 3.73 ± 0.45 |
| **Test t apparié Significativité** | t = 2.58 | t = 2.48 | t = 3.68 | t = 3.95 |
| | p < 0.05 | p < 0.05 | p < 0.01 | p < 0.01 |

Interprétation : Avant le coup de chaleur, les rats de tous les groupes ont discriminé le levier actif du levier inactif.

### Test d'évitement d'un stimulus lumineux aversif

Le TESLA a été réalisé à J19, trois jours après le coup de chaleur.

### Nombre total d'appuis sur les deux leviers

A l'issue des 20 minutes de test, l'ANOVA en mesures factorielles a montré que les nombres totaux d'appuis sur les deux leviers des rats des différents groupes ont été statistiquement équivalents (Tableau 15, Figure 14).

**Tableau 15 : Nombre total d'appuis sur les deux leviers au cours du test (Moyenne ± SEM).**

| | **TB (n = 16)** | **TCC (n = 15)** | **PP25 (n = 15)** | **PP50 (n = 15)** |
|---|---|---|---|---|
| **LA + LI** | 36.94 ± 7.41 | 30.00 ± 5.39 | 37.47 ±4.31 | 38.40 ± 6.61 |
| **ANOVA en mesures factorielles** | F _{(3,57)} = 0.39 | | | |
| | N.S. | | | |

Interprétation : Après le coup de chaleur, les rats des différents groupes ont montré la même activité manipulatoire.

### Discrimination entre les deux leviers

Pour évaluer la discrimination entre les leviers actif et inactif, seuls les appuis pendant la phase lumineuse ont été pris en compte. Seuls les rats ayant appuyé au moins une fois sur chaque levier ont été pris en compte dans l'analyse statistique.

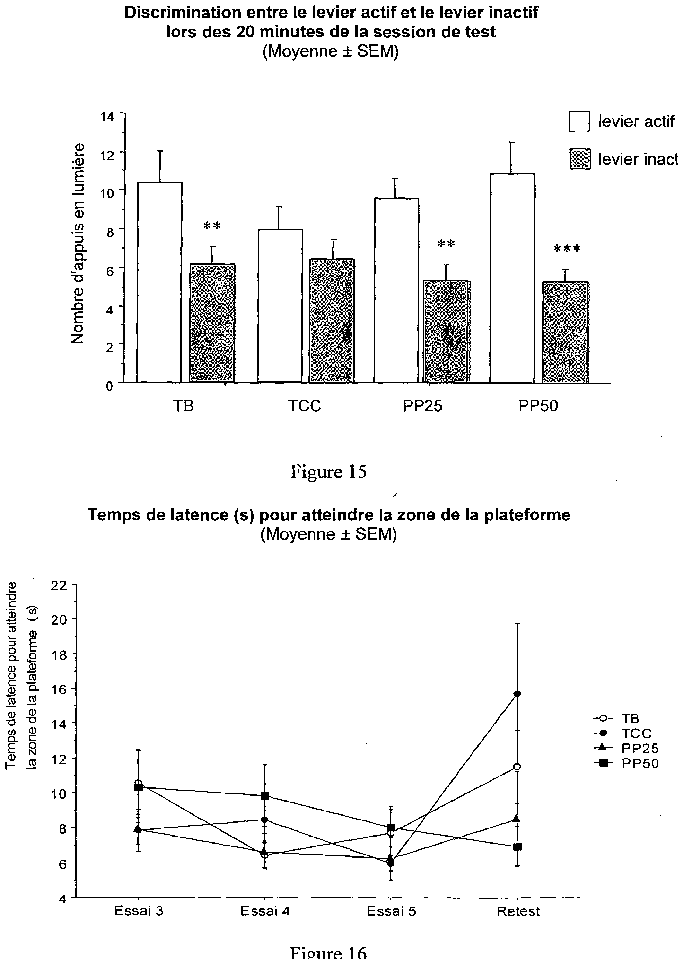

Pendant les 20 minutes de test, le test t apparié a montré que les rats des groupes TB, PP25 et PP50 ont discriminé significativement le levier actif du levier inactif, alors que les rats du groupe TCC n'ont pas montré de discrimination significative (Tableau 16, Figure 15).

**Tableau 16 : Discrimination entre les leviers actif et inactif lors des 20 minutes de la session de test (Moyenne ± SEM).**

| **Groupes** | **TB (n = 15)** | **TCC (n = 14)** | **PP25 (n = 14)** | **PP50 (n = 15)** |
|---|---|---|---|---|
| **LA** | 10.40 ± 1.67 | 7.93 ± 1.23 | 9.71 ± 1.00 | 10.93 ± 1.53 |
| **LI** | 6.13 ± 0.81 | 6.43 ± 1.00 | 5.64 ± 0.87 | 5.27 ± 0.75 |
| **Test t apparié Significativité** | t = 3.37 | t = 1.59 | t = 3.55 | t = 5.82 |
| | p < 0.01 | N.S. | p < 0.01 | p < 0.0001 |

Interprétation : Après le coup de chaleur, seuls les rats du groupe TCC ont appuyé de façon équivalente sur les deux leviers et n'ont donc plus montré de discrimination entre les deux leviers. Les rats exposés à la chaleur et traités aux polyphénols ont continué à discriminer le levier actif du levier inactif.

### Test du labyrinthe aquatique de Morris

Le test du labyrinthe aquatique de Morris a été réalisé à J20 et J21, 4 et 5 jours après le coup de chaleur.

Au niveau des essais 3, 4, 5, de la moyenne des essais 3, 4 et 5 et du retest, l'ANOVA en mesures factorielles n'a pas montré de différence significative au sein des temps de latence pour atteindre la zone de la plateforme des quatre groupes de rats (Tableau 16).

Entre la moyenne des essais 3, 4 et 5 et le retest, le test t apparié a montré une augmentation significative du temps de latence pour atteindre la zone de la plateforme pour les rats du groupe TCC. Une diminution significative du temps de latence pour atteindre la zone de la plateforme chez les rats du groupe PP50 et une stabilité chez les rats des groupes TB et PP25 ont été observées (Tableau 17, Figure 16).

**Tableau 17 : Temps de latence (s) pour atteindre la zone de la plateforme (Moyenne ± SEM)**

| | **TB (n = 16)** | **TCC (n = 15)** | **PP25 (n = 15)** | **PP50 (n = 15)** | **ANOVA en mesures factorielles** |
|---|---|---|---|---|---|
| **Essai 3** | 10.56 ± 1.96 | 7.87 ± 1.20 | 7.93 ± 0.85 | 10.33 ± 2.06 | F_{(3,57}) = 0.84 |
| | | | | | N.S. |
| **Essai 4** | 6.44 ± 0.71 | 8.47 ± 1.21 | 6.67 ± 1.01 | 9.87 ± 1.75 | F_{(3,51)} = 1.76 |
| | | | | | N.S. |
| **Essai 5** | 7.75 ± 1.29 | 6.00 ± 0.44 | 6.27 ± 1.21 | 8.07 ± 1.16 | F_{(3,57)} = 0.91 N.S. |
| **ANOVA en mesures répétées** | F_{(2,30)} = 2.86 | F_{(2,28)} = 2.03 | F_{(2.28)} = 0.79 | F_{(2,28)} = 0.48 | - |
| | p < 0.10 | N.S. | N.S. | N.S. | |
| **Moyenne des essais 3, 4 et 5 (Moy 3,4,5)** | 8.25 ± 0.98 | 7.44 ± 0.70 | 6.96 ± 0.66 | 9.42 ± 0.95 | F_{(3,57)} = 1.62 |
| | | | | | N.S. |
| **Retest** | 11.50 ± 2.09 | 15.73 ± 4.02 | 8.53 ± 2.70 | 7.00 ± 1.09 | F_{(3,57)} = 2.04 |
| | | | | | N.S. |
| **Test t apparié Moy 3,4,5 *vs.* Retest** | t = 1.53 | t = 2.37 | t = 0.56 | t = 2.18 | - |
| | N.S. | p < 0.05 | N.S. | p<0.05 | |

Interprétation : Le groupe exposé à la chaleur et non traité a présenté un déficit de mémoire à long terme alors que les groupes traités aux polyphénols n'ont pas présenté de déficit mnésique. Les rats du groupe traité à 50 mg/kg de polyphénols ont même amélioré leurs performances lors du retest.

### 4.4. Conclusions

Les résultats présentés plus haut montrent que l'administration en mode préventif des polyphénols de co-produits brassicoles aux doses de 25 et 50 mg/kg/j pendant 3 semaines a montré des effets protecteurs contre les perturbations cognitives chez les rats ayant subit un coup de chaleur.

### Exemple 5 : Exploration de l'activité d'un extrait riche en polyphénols sur les structures épidermiques et dermiques d'explants de peau humaine maintenus en survie

### 5.1. Objectif

L'objectif de l'étude présentée dans cet exemple était d'explorer les activités potentielles de différentes concentrations de polyphénols issus de la filière brassicole, en formulation ou incorporés dans les milieux de culture, sur les structures épidermiques et dermiques d'explants de peau humaine maintenus en survie.

Les activités ont été évaluées par une expertise histologique de la morphologie générale de la peau après coloration au trichrome de Masson, complétée (sur les lots présentant un intérêt particulier) par la visualisation des GAGs et par les immunomarquages des collagènes de type III et IV, et de la laminine-5.

### 5.2. Matériels et méthodes

### Produit testé

Le produit à tester était un extrait riche en polyphénols obtenu comme décrit dans l'exemple 2 ci-dessus. Il a été testé en application topique, incorporé dans de l'hydrocérine (une base pour préparations magistrales) à la concentration de 0.25 %, 0.50 %, 0.75 % et 1.00 %. Il a également été testé incorporé dans le milieu de culture, à raison de 0.025 %, 0.050 %, 0.075 % et 0.100 %. Deux autres produits ont été testés afin de servir de points de comparaison : une référence positive contenant du rétinol (Retin-Ox+ Nuit, RoC), et l'excipient (l'hydrocérine)

### Modèle ex vivo

Lors de la manipulation, des explants ont été préparés à partir de résidus de chirurgie plastique abdominale d'une femme caucasienne âgée de 44 ans. Le tissu adipeux a été éliminé, puis des explants d'un diamètre de 10 mm environ ont été préparés à l'aide d'un bistouri circulaire. Les explants ont ensuite été maintenus en survie dans des conditions classique de culture cellulaire pendant 10 jours.

Les explants ont été répartis en 15 lots de 6 explants et un lot témoin de 3 explants suivant la répartition ci-dessous :

**Tableau 18**

| **Lot** | | **Nombre d'explants** |
|---|---|---|
| **T0** | Témoin plastie | 3 explants |
| **T** | Témoin non traité | 6 explants |
| **R** | Référence positive (Retin-Ox+ Nuit) | 6 explants |
| **E** | Excipient (l'hydrocérine) | 6 explants |
| **P1T** | Application topique de 0.25 % de PPH-BK | 6 explants |
| **P2T** | Application topique de 0.50 % de PPH-BK | 6 explants |
| **P3T** | Application topique de 0.75 % de PPH-BK | 6 explants |
| **P4T** | Application topique de 1.00 % de PPH-BK | 6 explants |
| **P1M** | PPH-BK dans le milieu à 0.025 % | 6 explants |
| **P2M** | PPH-BK dans le milieu à 0.050 % | 6 explants |
| **P3M** | PPH-BK dans le milieu à 0.075 % | 6 explants |
| **P4M** | PPH-BK dans le milieu à 0.1 % | 6 explants |
| **P1TM** | P1T et P1M | 6 explants |
| **P2TM** | P2T et P2M | 6 explants |
| **P3TM** | P3T et P3M | 6 explants |
| **P4TM** | P4T et P4M | 6 explants |

Les produits à tester par voie topique ont été appliqués sur les explants à raison de 2 mg par explant (1 mg pour la référence positive) à J0, J2, J4, J6 et J8. Les produits à tester dans le milieu de culture y ont été incorporés aux concentrations indiquées. La moitié du milieu de culture a été renouvelée à J2, J4, J6 et J8.

A T0, les trois explants du lot témoin ont été prélevés, puis immédiatement coupés en deux. Une moitié a été fixée dans une solution de Bouin ordinaire, l'autre moitié a été congelée à -80°C. A J6 et J10, cette opération a été renouvelée pour trois explants de chaque lot.

Après 48 heures de fixation dans le Bouin ordinaire, les prélèvements ont été déshydratés et imprégnés en paraffine, puis mis en bloc à l'aide d'une station d'enrobage, afin de pouvoir procéder à la coupe. Des coupes de 5 µm d'épaisseur ont été réalisées et collées sur des lames de verre, en vue d'effectuer les colorations.

Des coupes de 7 µm d'épaisseur ont été réalisées à partir des prélèvements congelés, puis collées sur des lames de verre silanisées afin de réaliser les immunomarquages.

### Colorations - immunomarquages

L'observation de la viabilité cellulaire et de la morphologie générale a été effectuée sur des coupes colorées au trichrome de Masson. Cette coloration consiste à plonger les coupes dans des bains de colorants successifs, de façon à colorer spécifiquement certaines structures (les noyaux en noir, le cytoplasme des cellules en rose, le collagène en vert), ce qui permet de faciliter l'identification de ces structures lors de l'observation microscopique

L'observation des glucosaminoglycanes (GAGs) a été réalisée sur des coupes colorées au bleu alcian - P.A.S. Les GAGs sont des polysaccharides complexes retrouvés en abondance à la surface des cellules, et constituent un élément important des matrices extracellulaires. Les GAGs neutres, proche de la jonction dermo-épidermique (JDE) sont des réservoirs de facteurs de croissance ; les GAGs acides, localisés dans l'épiderme et le derme papillaire sont essentiellement constitués d'acide hyaluronique, impliqué dans l'hydratation de la peau. *In vivo,* les GAGs sont impliqués dans l'élasticité et l'hydratation de la peau. La coloration au bleu alcian P.A.S. permet de mettre en évidence l'expression des GAGs neutres le long de la JDE, sous la forme d'une bande rose violacée.

Les immunomarquages des collagènes III et IV ont été réalisés sur des coupes congelées. Les différents types de collagène sont des éléments majeurs des matrices extracellulaires. Le collagène III est un composant du derme fibrillaire, et le collagène IV est un composant de la JDE. La laminine-5 joue un rôle essentiel dans l'adhésion des kératinocytes à la membrane basale en participant à la formation des complexes d'ancrage. *In vivo,* ils sont impliqués dans le tonus et la résistance mécanique de la peau.

L'immunomarquage du collagène III a été révélé par la DAB, qui montre une coloration marron. Une contre-coloration par l'hémalun de Masson a été effectuée sur les noyaux des cellules afin de les faire apparaître en bleu. L'immunomarquage du collagène IV et de la laminine-5 ont été révélés par la FITC, une molécule fluorescente qui lorsqu'elle est excitée, émet une lumière verte. Une contre-coloration à l'iodure de propidium a été effectuée sur les noyaux des cellules afin de les faire apparaître en rouge.

### Observations microscopiques

Les observations microscopiques ont été réalisées en microscopie optique, à l'aide d'un microscope à l'objectif x 40. Les prises de vue ont été réalisées avec une caméra numérique et stockées à l'aide d'un logiciel d'archivage.

### 5.3. Résultats et discussion

Seuls les résultats les plus significatifs sont présentés ici.

### Observation microscopique

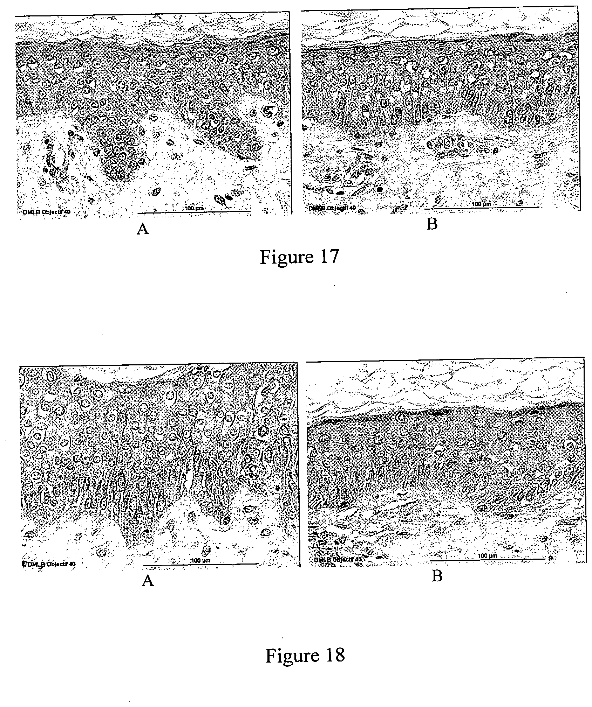

A J0, la morphologie générale des explants est normale. A J6, la morphologie des explants non traités est comparable à celle des explants à J0, ce qui est un bon indicateur de la survie des explants (Figure 17).

Sur les explants traités avec la formulation de référence contenant du rétinol, l'acanthose (augmentation de l'épaisseur épidermique) est très nette. Sur les explants traités avec l'excipient, les structures épidermique et dermique sont proches de celles observées sur les explants non traités (Figure 18).

Sur les explants traités avec les formulations P2T et P2M, l'acanthose épidermique est nette, mais moindre que pour les explants traités avec la référence contenant du rétinol. Pour tous les autres explants, la structure épidermique est proche de celle des explants traités avec l'excipient. En ce qui concerne le réseau de collagène dans le derme papillaire, il apparaît :
- très dense sur les explants du lot P1TM ;
- dense, notamment le long de la jonction dermo-épidermique sur les explants des lots P2TM et P4TM ;
- assez dense sur les explants des lots P4T, P2M, P3M, P4M et P3TM ;
- proche de celui observé sur les explants traités avec l'excipient sur ceux des lots P2T, P3T et P1M.

Les plus nettes observations ont été réalisées après 6 jours de survie.

A J10, sur les explants non traités, la structure épidermique est normale. Le derme papillaire est plus ou moins dense. Sur les explants traités avec la formulation de référence contenant du rétinol, l'acanthose épidermique est très nette. Dans le derme papillaire, le collagène forme un réseau plus ou moins dense le long de la jonction dermo-épidermique. Sur les explants traités avec l'excipient, la structure épidermique et dermique est proche de celle observée sur les explants non traités.

Sur les explants traités avec la formulation P4TM, l'épiderme est nettement acanthosique, mais avec de très nets aspects d'intolérance. Pour les autres produits testés, la structure épidermique est proche de celle des explants traités avec l'excipient. En ce qui concerne le réseau de collagène dans le derme papillaire, il apparaît :
- dense dans le derme papillaire sur les explants du lot P2M ;
- assez dense sur les explants traités avec les produits P2T, P3T, P4T, P3M et P4TM ;
- plus ou moins dense, proche de celui observé sur les explants non traités avec les produits P1M, P4M, P1TM, P2TM et P3TM.

L'ensemble des paramètres évalués lors de l'observation microscopique de la morphologie a permis de sélectionner deux lots sur lesquels la suite de l'évaluation de l'activité a été pratiquée. Ces lots répondent à plusieurs exigences :
- innocuité :
   ∘ absence d'intolérance et d'altérations cellulaires ;
- efficacité :
   ∘ augmentation de l'épaisseur de l'épiderme ;
   ∘ amélioration de la compacité du réseau de collagène dans le derme papillaire.
- coût :
   ∘ faible concentration

### Coloration des GAGs

Sur les explants non traités, les GAGs neutres sont assez modérément visualisés le long de la jonction dermo-épidermique. Ils sont modérément surexprimés sur les explants traités avec la référence positive.

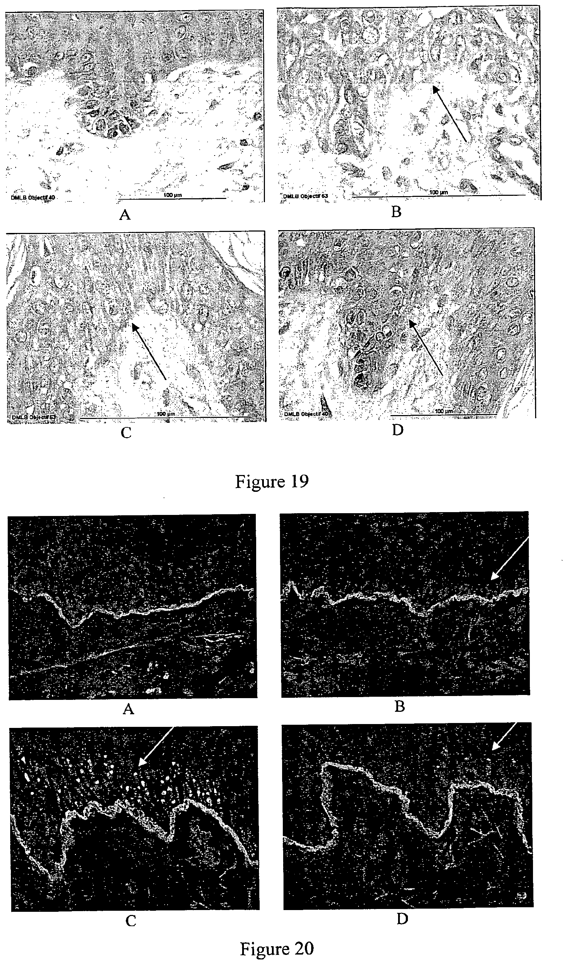

L'application en topique du produit PPH-BK à 0,5 % induit une surexpression modérée. L'incorporation du produit PPH-BK à 0,025 % dans le milieu de survie induit une nette surexpression (Figure 19).

Ces résultats indiquent que le produit PPH-BK, appliqué par voie topique à la concentration de 0,5 %, induit une amélioration de l'un des paramètres associé à l'hydratation de la peau comparable à celle induite par la référence positive. Lorsqu'il est incorporé dans le milieu à la concentration de 0,025 %, cette amélioration est nettement supérieure.

### Immunomarquage de la laminine-5

Sur les explants non traités, la laminine-5 est nettement visualisée formant une bande fine et festonnée le long de la jonction dermo-épidermique. Elle est légèrement surexprimée sur les explants traités avec la référence positive.

L'application en topique du produit PPH-BK à 0,5 % induit une surexpression modérée le long de la jonction dermo-épidermique et nette dans les kératinocytes basaux. L'incorporation du produit PPH-BK à 0,025 % dans le milieu de survie induit une légère surexpression au niveau de la jonction dermo-épidermique et des kératinocytes basaux (figure 20).

Ces résultats indiquent que le produit PPH-BK, appliqué par voie topique à la concentration de 0,5 %, induit une augmentation de l'expression très supérieure à celle induite par la référence positive. Lorsqu'il est incorporé dans le milieu à la concentration de 0,025 %, l'augmentation de l'expression est légèrement supérieure à celle de la référence.

### Immunomarquage du collagène III

Sur les explants non traités, le collagène III est légèrement visualisé formant un réseau peu dense dans le derme papillaire, le long de la jonction dermo-épidermique. Il est modérément surexprimé sur les explants traités avec la référence positive.

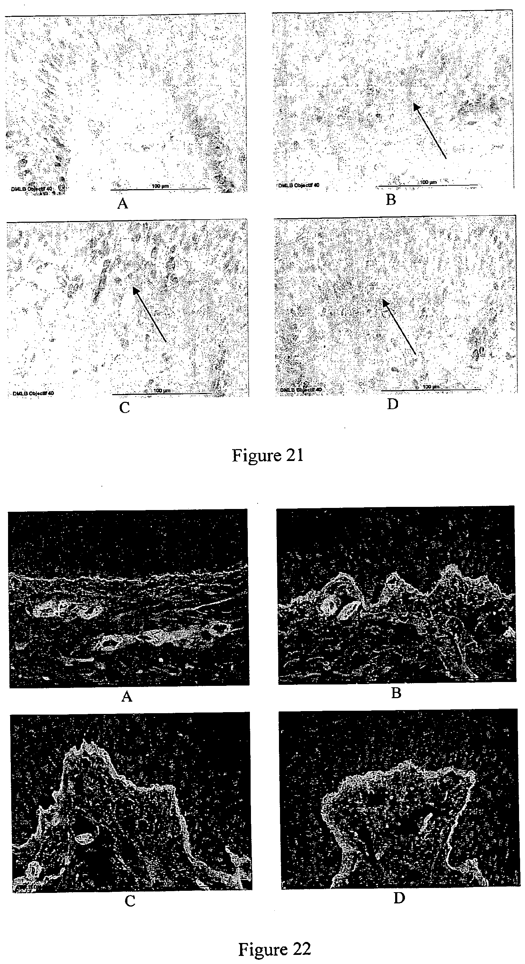

L'application en topique du produit PPH-BK à 0,5 % induit une nette surexpression. L'incorporation du produit PPH-BK à 0,025 % dans le milieu de survie induit une légère surexpression (Figure 21).

Ces résultats indiquent que le produit PPH-BK, appliqué par voie topique à la concentration de 0,5 %, induit une augmentation de l'expression supérieure à celle induite par la référence positive. Lorsqu'il est incorporé dans le milieu à la concentration de 0,025 %, l'augmentation de l'expression est comparable à celle de la référence.

### Immunomarquage du collagène IV

Sur les explants non traités, le collagène IV est nettement visualisé le long de la jonction dermo-épidermique. Il est peu régulier et modérément présent dans le derme papillaire sous-jacent. Il est nettement surexprimé sur les explants traités avec la référence positive.

L'application en topique du produit PPH-BK à 0,5 % induit une nette surexpression, surtout le long de la jonction dermo-épidermique. L'incorporation du produit PPH-BK à 0,025 % dans le milieu de survie induit une nette surexpression, surtout le long de la jonction dermo-épidermique (Figure 22).

Ces résultats indiquent que le produit PPH-BK, appliqué par voie topique à la concentration de 0,5 % ou incorporé dans le milieu à la concentration de 0,025 %, induit une augmentation de l'expression comparable à celle induite par la référence positive.

### 5.4. Conclusion

Les résultats sont résumés dans le tableau ci-après.

**Tableau 19**

| **Lot** | **Nombre d'assises cellulaires épidermiques** | | **Morphologie épidermique** | | **Densité du collagène** | |
|---|---|---|---|---|---|---|
| | **J6** | **J10** | **J6** | **J10** | **J6** | **J10** |
| T | 4/5 | 5/6 | OK | OK | +/- dense | +/- dense |
| R | 13/14 | 13/14 | Oedème | Fort oedème | +/- dense | +/- dense |
| E | 5/6 | 4/5 | OK | OK | +/- dense | Peu dense |
| P2T | 7/8 | ¾ | OK | OK | +/- dense | Assez dense |
| P3T | 5/6 | 4/5 | OK | OK | +/-dense | Assez dense |
| P4T | 7/8 | 4/5 | OK | OK | Assez dense | Assez dense |
| P1M | 5/6 | 5/6 | OK | OK | +/-dense | +/- dense |
| P2M | 4/5 | 4/5 | OK | OK | Assez dense | dense |
| P3M | 5/6 | 4/5 | OK | OK | Assez dense | Assez dense |
| P4M | 4/5 | 4/5 | OK | OK | Assez dense | +/ |
| P1TM | 5/5 | 4/5 | OK | OK | Très dense | +/ |
| P2TM | 5/6 | 4/5 | OK | OK | Dense | +/ |
| P3TM | 5/6 | 4/5 | Assez bonne | Assez bonne | Assez dense | +/ |
| P4TM | 5/6 | 7/8 | altérée | Très altérée | Assez dense | Assez dense |

Ces résultats montrent que par les deux modes d'application testés (application topique ou incorporation dans le milieu), le produit induit une augmentation de l'expression de plusieurs paramètres associés à l'hydratation, l'élasticité et la jeunesse de la peau, dès 6 jours. Par ailleurs, son effet sur ces paramètres est comparable ou supérieur à celui d'une crème de référence contenant du rétinol.

Ceci indique que les polyphénols extraits du procédé de brassage peuvent être utiles en cosmétique, au moins dans les applications suivantes :
- anti-oxydant
- anti-âge (augmentation de l'épaisseur de l'épiderme)
- hydratation.

### Exemple 6 : Composition des extraits de polyphénols obtenus

### 6.1. Evaporation des échantillons

### But des essais

- Observer le comportement des produits en cours d'évaporation et déterminer leur retard à l'ébullition.
- Déterminer les coefficients d'échange aux différentes étapes de concentration.
- Observer leur capacité à se concentrer et prévoir la qualité des condensats.

### Mode préparatoire

Les essais ont été réalisés sur un évaporateur industriel type flot tombant, sous faible dépression (P = 0.25 bar abs, Téq = 65°C), en batch.

Les essais ont été réalisés en 3 batchs distincts : A, B, C. Chaque batch représentant un volume de V = 100 L.

### Caractéristiques de l'affluent

∘ Matière sèche produit brut 1.5 %
∘ Description Noir avec forte odeur d'alcool

### Comportement durant l'évaporation

### Batch A :

- Début de la concentration : Volume cubitainer = 100 L
- Fin de la concentration : Volume dans l'évaporateur = 15 L
- Le facteur de concentration volumique obtenu est donc de 6,6.
- Temps de séjour dans l'évaporateur : 2h
- A la fin de l'essai, on constate un dépôt sur le haut des parois de l'évaporateur.

Cependant celui ci se nettoie très bien à l'aide d'une solution d'alcool.

### Batch B :

- Début de la concentration : Volume cubitainer = 95L
- Fin de la concentration : Volume dans l'évaporateur = 25 L
- Le facteur de concentration volumique obtenu est donc de 3,8.
- Temps de séjour dans l'évaporateur : 1h40
- A la fin de l'essai, on constate qu'il n'y a pas de dépôt sur le haut des parois de l'évaporateur. Le concentré conserve une odeur d'alcool.

### Batch C:

- Début de la concentration : Volume cubitainer = 100 L
- Fin de la concentration : Volume dans l'évaporateur = 30 L
- Le facteur de concentration volumique obtenu est donc de 3,3.
- Temps de séjour dans l'évaporateur : 1h45
- A la fin de l'essai, on constate qu'il n'y a pas de dépôt sur le haut des parois de l'évaporateur. Le concentré conserve une odeur d'alcool.

### 6.2. Analyse des échantillons

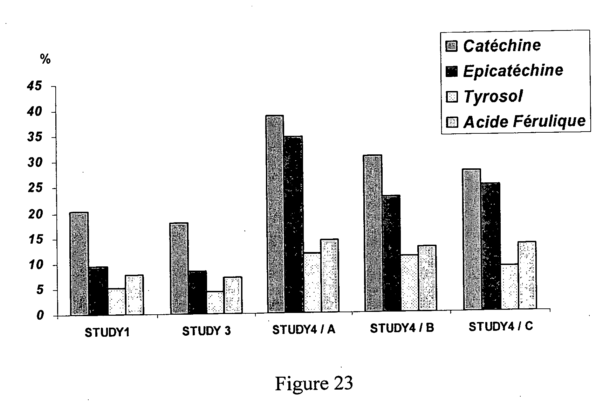

Les échantillons obtenus par évaporation ont ensuite été lyophilisés et analysés par chromatographie liquide à haute performance (HPLC). Les résultatas sont présentés dans les tableaux ci-dessous, ainsi qu'à la figure 23.

**Tableau 20 : analyse des échantillons lyophilisés**

| | | Powder A | Powder B | Powder C |
|---|---|---|---|---|
| | Fer | 0 | 0 | 0 |
| | Cuivre | 0,003 | 0,003 | 0,0005 |
| | Calcium | 2,848 | 1,094 | 1,227 |
| | Magnésium | 0,428 | 0,231 | 0,263 |
| | Potassium | 0,436 | 0 | 0 |
| | Sodium | 3,469 | 0,585 | 0,687 |
| | Zinc | 0,011 | 0,009 | 0,007 |
| mg/g | Chlorures (Cl) | 5,73 | 0,609 | 0,4 |
| | Sulphates | 1,555 | 0,557 | 0,801 |
| | Nitrates | 0 | 0 | 0 |
| | Acide oxalique libre | 1,687 | 0 | 0,233 |
| | Plomb | 0 | 0 | 0 |
| | Cadmium | 0 | 0 | 0 |
| | Chrome | 0 | 0 | 0 |
| | Arsenic | 0 | 0 | 0 |
| | Aluminium | 0,002 | 0,002 | 0,002 |
| | Nitrure de titane | 0 | 0 | |
| | Cobalt | 0 | 0 | 0 |
| | Mercure | 0 | 0 | 0 |
| | Manganèse | 0 | 0 | 0 |
| | Phosphore | 1,73 | 1,66 | 1,8 |
| | Nickel | 0 | 0 | 0 |
| | Total Mineraux mg/g | 17,899 | 4,75 | 5,4205 |
| | **Total Minéraux %** | **1,7899** | **0,475** | **0,54205** |
| | | | | |
| | Total sucres mg/g | 0,47 | 0,33 | 0,33 |
| | **total sucres %** | **0,047** | **0,033** | **0,033** |
| | | | | |
| | Total protéines % / WM | 1,1 | 1,8 | 1,8 |
| | **Humidité %** | **1** | **2,1** | **1,8** |
| | Polyphénols mg/g | 697 | 735 | 865 |
| | Méthode EBC | | | |
| | **Polyphénols EBC %** | **69,7** | **73,5** | **86,5** |
| | Polyphénols mg/g | | | |
| | équ.ac.gallique | 320 | 338 | 399 |
| | Polyphénols Folin-Ciocalteu | 478 | 529 | 483 |
| | mg/G équ.ac.gallique | | | |
| | % | **47,8** | **52,9** | **48,3** |
| | poudre anti-oxydant DDPH mg/g de polyphénols (EBC) | 3796 | 3533 | 3244 |
| après hydrolyse | Fructose (PN) | 0 | 0 | 0 |
| | Glucose (PN) | 0 | 0 | 0 |
| | Saccharose (PN) | 0 | 0 | 0 |
| | Maltose (PN) | 0 | 0 | 0 |
| | Maltotriose (P/V) | 0 | 0 | 0 |

**Tableau 21 : composition en flavonols et acides phénoliques (% masse).**

| | A | B | C |
|---|---|---|---|
| ***Catéchine*** | 38,75 | 30,77 | 27,68 |
| ***Epicatéchine*** | 34,62 | 22,70 | 24,83 |
| ***Tyrosol*** | 11,79 | 11,13 | 8,86 |
| ***Acide Férulique*** | 14,30 | 12,74 | 13,10 |
| ***Total % flavanols+Ac.férulique*** | 99,46 | 77,34 | 74,46 |

**Tableau 22 : bilan massique**

| | A | B | C |
|---|---|---|---|
| ***flavanols et a.phénoliques totaux*** | 99,46 | 77,34 | 74,46 |
| ***protéines totaesl*** | 1,10 | 1,80 | 1,80 |
| ***minéraux*** | 1,79 | 0,48 | 0,54 |
| ***sucres*** | 0,05 | 0,03 | 0,03 |
| ***humidité*** | 1,00 | 2,10 | 1,80 |
| ***Total*** | 103,40 | 81,75 | 78,64 |

### REFERENCES

Blokland A, Geraerts E, Been M. A detailed analysis of rats' spatial memory in a probe trial of a Morris task. Behavioral Brain Research. 2004, 154(1): 71-5.
Brand-Williams W., Cuvelier M. E. and Berset C., Use of a Free Radical Method to Evaluate Antioxidant Activity. Lebensm. Wiss. Technol., 1995, 28(1), 25-30.
Brouillard R., George F. and Fougerousse A., Polyphenols produced during red wine ageing. BioFactors, 1997, 6(4), 403-410.
Canini F, Buguet A, Bourdon L. Inhibition of different types of nitric oxide synthase: effect on thermoregulation in the rat exposed to high ambient temperature. Neurosciences Letters, 2001, 316(1): 45-9.
Folin O. and Ciocalteu V., On Tyrosine and Tryptophane Déterminations in Proteins. J. Biol. Chem., 1927, 73(2), 627-650.
McMurrough I., Roche G. P. and Cleary K. G., Phenolic acids in beers and worts. J. Inst. Brew., 1984, 90(3), 181-187.
Messaoudi M, Tricoire A, Lalonde R, Canini F, Minn A. Effects of MPTP on lever-pressing for light extinction in rats. European Journal of Pharmacology, 1996, 299: 17-20.
Messaoudi M, Desor D, Grasmück V, Joyeux M, Langlois A, Roman FJ. Behavioral evaluation of visceral pain in a rat model of colonic inflammation. Neuroreport, 1999, 10: 1137-1141.
Morris RG, Garrud P, Rawlins JN, O'Keefe J. Place navigation impaired in rats with hippocampal lesions. Nature, 1982, 297(5868): 681-3.
Prost, M. Process for the determination by means of free radicals on the antioxidant properties of a living organism or potentially aggressive agents. US Patent, 1992, 5, 135, 850.
Tubaro F., Rapuzz i P. and Ursini A. F., Kinetic analysis of antioxidant capacity of wine BioFactors, 1999, 9(1), 37-47.
Yang CY, Lin MT. Oxidative stress in rats with heatstroke-induced cerebral ischemia. Stroke, 2002, 33: 790-794.

## Revendications

1. Procédé d'obtention d'un extrait de polyphénols issus du procédé de brassage, comprenant au moins les étapes suivantes :
(i) mise en contact d'une bière partiellement purifiée, obtenue après élimination des levures, débris végétaux et particules colloïdales, avec une résine adsorbant les polyphénols ;
(ii) récupération des polyphénols adsorbés sur ladite résine,
l'étape (ii) comportant une étape de désorption des polyphénols, à l'aide d'un solvant organique, d'une résine hydrophobe et non ionique adsorbant les polyphénols.

2. Procédé selon la revendication 1, dans lequel l'étape de récupération des polyphénols comprend les étapes suivantes :
(i) désorption des polyphénols adsorbés sur la résine mise au contact de la bière partiellement purifiée ;
(ii) adsorption des polyphénols sur une deuxième résine distincte de la première, ladite deuxième résine étant hydrophobe et non ionique ; et
(iii) désorption des polyphénols adsorbés sur la deuxième résine, à l'aide d'un solvant organique.

3. Procédé selon la revendication 1, dans lequel la bière partiellement purifiée est mise au contact d'une résine hydrophobe et non ionique adsorbant les polyphénols.

4. Procédé selon les revendications 1 et 2, comportant les étapes suivantes :
(i) la bière partiellement filtrée est mise au contact d'une résine de polyvinylpolypyrrolidone (PVPP) ;
(ii) la PVPP est lavée avec une solution alcaline ;
(iii) la solution de lavage de la PVPP est mise au contact d'une deuxième résine adsorbant les polyphénols, ladite deuxième résine étant hydrophobe et non ionique ;
(iv) les polyphénols sont désorbés de la deuxième résine ;
(v) les polyphénols sont concentrés.

5. Procédé selon la revendication 4, dans lequel, à l'étape (ii), la PVPP subit deux lavages à la soude, et dans lequel seule la solution de soude du premier lavage est utilisée à l'étape (iii).

6. Procédé selon la revendication 4 ou la revendication 5, dans lequel la solution alcaline utilisée pour le lavage de la PVPP est neutralisée ou acidifiée avant la mise en oeuvre de l'étape (iii).

7. Procédé selon l'une quelconque des revendications 4 à 6, dans lequel la deuxième résine utilisée est une résine polymérique adsorbante, aromatique, hydrophobe et non ionique.

8. Procédé selon l'une quelconque des revendications 4 à 7, dans lequel la deuxième résine est rincée avec une solution aqueuse entre les étapes (iii) et (iv).

9. Procédé selon la revendication 3, dans lequel la bière partiellement filtrée est mise au contact d'une résine adsorbant les polyphénols et permettant leur désorption par un solvant organique, ladite résine étant une résine polymérique adsorbante, aromatique, hydrophobe et non ionique.

10. Concentré de polyphénols susceptible d'être obtenu par un procédé selon l'une quelconque des revendications 1 à 9.

11. Complément alimentaire comprenant un concentré de polyphénols selon la revendication 10, **caractérisé en ce qu'**il se présente sous forme de gélules, de poudre ou de comprimés.

12. Utilisation d'un produit cosmétique comprenant un concentré de polyphénols selon la revendication 10, pour hydrater la peau et/ou prévenir ou ralentir son vieillissement.

13. Utilisation d'un concentré de polyphénols selon la revendication 10, pour la préparation d'une composition anti-oxydante.

14. Utilisation selon la revendication 13, pour la préparation d'une composition destinée à améliorer les performances cognitives d'un individu.

15. Utilisation selon la revendication 13, pour la préparation d'une composition anti-vieillissement.

16. Utilisation selon la revendication 14 ou la revendication 15, pour limiter ou retarder les conséquences du vieillissement cérébral.

## Patentansprüche

1. Verfahren zur Gewinnung eines Polyphenolextrakts aus einem Brauverfahren, das mindestens die folgenden Schritte umfasst:
(i) Inkontaktbringung eines teilweise gereinigten Bieres, das nach Beseitigung der Hefen, Pflanzenreste und kolloidalen Partikel erhalten wird, mit einem die Polyphenole adsorbierenden Harz;
(ii) Rückgewinnung der auf dem Harz adsorbierten Polyphenole,
wobei der Schritt (ii) einen Schritt zur Desorption von den Polyphenolen von einem wasserabweisenden und nichtionischen Harz, das die Polyphenole adsorbiert, mittels eines organischen Lösungsmittels umfasst.

2. Verfahren nach Anspruch 1, wobei der Schritt zur Rückgewinnung der Polyphenole die folgenden Schritte umfasst:
(i) Desorption der auf dem mit dem teilweise gereinigten Bier in Kontakt gebrachten Harz adsorbierten Polyphenole;
(ii) Adsorption der Polyphenole auf einem zweiten Harz, das sich von dem ersten unterscheidet, wobei das zweite Harz wasserabweisend und nichtionisch ist; und
(iii) Desorption der auf dem zweiten Harz adsorbierten Polyphenole mittels eines organischen Lösungsmittels.

3. Verfahren nach Anspruch 1, wobei das teilweise gereinigte Bier mit einem hydrophoben und nichtionischen Harz in Kontakt gebracht wird, das die Polyphenole adsorbiert.

4. Verfahren nach Anspruch 1 und 2, das die folgenden Schritte umfasst:
(i) das teilweise filtrierte Bier wird mit einem Polyvinylpolypyrrolidon-Harz (PVPP) in Kontakt gebracht;
(ii) das PVPP wird mit einer alkalischen Lösung gewaschen;
(iii) die Lösung zum Waschen des PVPP wird mit einem zweiten Harz in Kontakt gebracht, das die Polyphenole adsorbiert, wobei das zweite Harz wasserabweisend und nichtionisch ist;
(iv) die Polyphenole werden von dem zweiten Harz desorbiert;
(v) die Polyphenole werden konzentriert.

5. Verfahren nach Anspruch 4, wobei im Schritt (ii) das PVPP zwei Waschungen mit Natriumkarbonat unterzogen wird und wobei einzig die Natriumkarbonatlösung der ersten Waschung im Schritt (iii) verwendet wird.

6. Verfahren nach Anspruch 4 oder Anspruch 5, wobei die alkalische Lösung, die zur Waschung des PVPP verwendet wird, vor der Durchführung von Schritt (iii) neutralisiert oder angesäuert wird.

7. Verfahren nach einem der Ansprüche 4 bis 6, wobei das zweite Harz, das verwendet wird, ein adsorbierendes, aromatisches, wasserabweisendes und nichtionisches Polymerharz ist.

8. Verfahren nach einem der Ansprüche 4 bis 7, wobei das zweite Harz zwischen den Schritten (iii) und (iv) mit einer wässrigen Lösung gespült wird.

9. Verfahren nach Anspruch 3, wobei das teilweise filtrierte Bier mit einem Harz in Kontakt gebracht wird, das die Polyphenole absorbiert und ihre Desorption durch ein organisches Lösungsmittel ermöglicht, wobei das Harz ein adsorbierendes, aromatisches, wasserabweisendes und nichtionisches Polymerharz ist.

10. Polyphenolkonzentrat, das durch ein Verfahren nach einem der Ansprüche 1 bis 9 erhalten werden kann.

11. Nahrungsergänzungsmittel, das ein Polyphenolkonzentrat nach Anspruch 10 umfasst, **dadurch gekennzeichnet, dass** es die Form von Kapseln, Pulver oder Tabletten aufweist.

12. Verwendung eines Kosmetikprodukts, das ein Polyphenolkonzentrat nach Anspruch 10 umfasst, zur Hydratisierung der Haut und/oder zur Vorbeugung oder Verlangsamung ihrer Alterung.

13. Verwendung eines Polyphenolkonzentrats nach Anspruch 10 zur Zubereitung einer antioxidierenden Zusammensetzung.

14. Verwendung nach Anspruch 13 zur Herstellung einer Zubereitung, die dazu bestimmt ist, die kognitiven Leistungen eines Individuums zu verbessern.

15. Verwendung nach Anspruch 13 zur Herstellung einer Antialterungszusammensetzung.

16. Verwendung nach Anspruch 14 oder Anspruch 15 zur Begrenzung oder Verzögerung der Folgen der Hirnalterung.

## Claims

1. A method for obtaining polyphenol extracts from a brewing process, comprising the following steps:
(i) putting a partly purified beer, obtained after removal of yeasts, plant debris and colloidal particles, in contact with a resin adsorbing polyphenols;
(ii) recovering polyphenols adsorbed on said resin,
the step (ii) including a step for desorption of the polyphenols, by means of an organic solvent, from a hydrophobic and non-ionic resin absorbing polyphenols.

2. The method according to claim 1, wherein the step for recovering polyphenols comprises the following steps;
(i) desorption of the polyphenols adsorbed on the resin put into contact with the partly purified beer;
(ii) adsorption of the polyphenols on a second resin distinct from the first, said second resin being hydrophobic and non-ionic; and
(iii) desorption of the polyphenols adsorbed on the second resin, by means of an organic solvent.

3. The method according to claim 1, wherein the partly purified beer is put into contact with a hydrophobic and non-ionic resin adsorbing polyphenols.

4. The method according to claims 1 and 2, including the following steps:
(i) the partly filtered beer is put into contact with a polyvinylpolypyrrolidone (PVPP) resin;
(ii) the PVPP is washed with an alkaline solution;
(iii) the washing solution of PVPP is put into contact with a second resin adsorbing polyphenols, said second resin being hydrophobic and non-ionic;
(iv) the polyphenols are desorbed from the second resin;
(v) the polyphenols are concentrated.

5. The method according to claim 4, wherein, in step (ii), the PVPP undergoes two washes with soda, and wherein only the soda solution of the first wash is used in step (iii).

6. The method according to claim 4 or claim 5, wherein the alkaline solution used for washing PVPP is neutralized or acidified before applying step (iii).

7. The method according to any of claims 4 to 6, wherein the second resin used is an adsorbing, aromatic, hydrophobic and non-ionic polymeric resin.

8. The method according to any of claims 4 to 7, wherein the second resin is rinsed with an aqueous solution between steps (iii) and (iv).

9. The method according to claim 3, wherein the partly filtered beer is put into contact with a resin adsorbing polyphenols and allowing their desorption with an organic solvent, said resin being an adsorbing, aromatic, hydrophobic and non-ionic polymeric resin.

10. A concentrate of polyphenols which may be obtained by a method according to any of claims 1 to 9.

11. A food supplement comprising a concentrate of polyphenols according to claim 10, **characterized in that** it appears as gelatin capsules, powder or tablets.

12. The use of a cosmetic product comprising a concentrate of polyphenols according to claim 10, for moisturizing the skin or preventing and/or slowing down ageing.

13. The use of a concentrate of polyphenols according to claim 10, for preparing an anti-oxidation composition.

14. The use according to claim 13, for preparing a composition intended for improving the cognitive performances of an individual.

15. The use according to claim 13, for preparing an anti-ageing composition.

16. The use according to claim 14 or claim 15, for limiting or delaying the consequences of ageing of the brain.
